(19) Europäisches Patentamt
European Patent Office
Office européen des brevets

(11) EP 1 200 046 B1

(12) **EUROPEAN PATENT SPECIFICATION**

(45) Date of publication and mention of the grant of the patent:
**27.10.2004 Bulletin 2004/44**

(21) Application number: **00953937.0**

(22) Date of filing: **10.08.2000**

(51) Int Cl.[7]: **A61K 7/06**, A61K 7/48, A61K 7/11, A61K 7/15, A61K 7/043, A61K 7/02, A61K 7/032

(86) International application number:
**PCT/US2000/021872**

(87) International publication number:
**WO 2001/010397 (15.02.2001 Gazette 2001/07)**

(54) **COSMETICS CONTAINING AMPHOTERIC POLYURETHANES**

KOSMETIKA ENTHALTEND AMPHOTHERE POLYURETHANE

COSMETIQUES CONTENANT DES POLYURETHANNES AMPHOTERES

(84) Designated Contracting States:
**AT BE CH CY DE DK ES FI FR GB GR IE IT LI LU MC NL PT SE**

(30) Priority: **10.08.1999 JP 22655899**

(43) Date of publication of application:
**02.05.2002 Bulletin 2002/18**

(73) Proprietor: **National Starch and Chemical Investment Holding Corporation**
**Wilmington, Delaware 19809-7663 (US)**

(72) Inventors:
- **ASAOKA, Seiji**
  **Suita-shi, Osaka 565-0841 (JP)**
- **KOYAMA, Katsuya**
  **Itami-shi, Hyogo 664-0898 (JP)**
- **TSUZUKI, Toshitaka**
  **Minoo-shi, Osaka 562-0036 (JP)**
- **HASHIMOTO, Tomohiro**
  **Kawanishi-shi, Hyogo 666-0138 (JP)**

(74) Representative:
**Held, Stephan, Dr.rer.nat., Dipl.-Chem. et al**
**Meissner, Bolte & Partner**
**Postfach 86 03 29**
**81630 München (DE)**

(56) References cited:
**EP-A- 0 775 483**
**EP-A- 0 793 957**
**US-A- 6 126 929**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 200 046 B1

## Description

Detailed Description of the Invention

The present invention relates to cosmetics containing amphoteric urethane resin and water-soluble resin.
It is customary to use water-soluble resins such as nonionic polyvinyl pyrrolidone (PVP), cationic acrylic resin or cellulose, anionic acrylic resin or polyvinyl acetate, amphoteric acrylic resin or polyvinyl acetate etc. as a base resin of cosmetics such as hair fixative. The hair fixative using these water-soluble resins as base resin is advantageous in that it has a high curl retention and excellent durability.
However, in order to obtain hair fixative which is excellent in durability as above-mentioned, it is necessary to use a water-soluble resin which has a high glass-transition temperature (Tg), which has a drawback in that its feel, which is important for cosmetics, is not good and its touch is very poor.
The present invention has been made in view of such circumstance with the object of providing cosmetics which include antithetic physical properties, i.e. touch and durability.
In order to attain the said object, the cosmetics of the present invention have a structure that contains amphoteric resin having carboxyl group(s) and tertiary amino group(s) in one molecule and water-soluble resin.
The inventors of the present invention have found that in the hair fixative having as a base resin amphoteric urethane resin containing carboxyl group(s) and tertiary amino group(s) in one molecule, originally antithetic physical properties such as setting ability and touch, combing ability and resistance to flaking are compatible with each other by the elasticity and toughness that the urethane resin possesses. Furthermore, they have also found that for neutral water, the said hair dressing is excellent in resistance to water due to the ionic bonding of the carboxyl group(s) and the tertiary amino group(s), and, for surface-active agent solutions such as shampoo in hair washability, due to dissociation of the said ionic bonding, and at the same time, the said cationic tertiary amino group(s) exhibit(s) a good adherence resulting from its interaction with the surface of negatively charged hair, and they have filed a patent application for the cosmetic resin compositions containing the said amphoteric urethane resin as the principal ingredient (Japanese Patent Application No.H10-27595). Thus, the amphoteric urethane resin has extremely excellent physical properties, but on the other hand, there lies a problem in durability, and they ,(the said inventors), have repeated studies for the purpose of improvement of the durability of amphoteric urethane resin. As a consequence, they have discovered that if water-soluble resin and amphoteric urethane resin are used together, the problem of the durability which is a weak point of amphoteric urethane resin can be solved by the use of water-soluble resin, and the problem of the touch which is a weak point of water-soluble resin can be solved by the use of amphoteric resin, thus resulting in the obtaining of a cosmetic rendered with antithetic physical properties such as touch and durability, and they have attained to the present invention.
Additionally the durability may be further improved by using nonionic resin, anionic resin ,cationic resin or amphoteric resin as said water-soluble resins.
The introduction into the structure of the said amphoteric urethane resin of the structural unit which may be derived from ethylene oxide as a nonionic hydrophilic constituent, may provide sufficient hydrophilic nature, which ensures improvement of hair washability especially when it is used as hair cosmetic material.
Additionally, the introduction of polysiloxane bond(s) into the structure of the said amphoteric urethane resin may improve the feel which may be felt especially when it is used as hair cosmetic material.

EXAMPLES

Next, the Examples of the present invention will be described.
The cosmetic materials in accordance with the present invention may be obtained by using amphoteric urethane resin containing carboxyl group(s) and tertiary amino group(s) in one molecule, and, water-soluble resin.
The cosmetics in accordance with the present invention may be used as hair cosmetics such as foam hair fixative, gel-like hair fixative, aerosol spray hair fixative, pump spray hair fixative, or cream-like hair fixative; skin care cosmetics such as shaving cream, skin care lotion, or sun screen lotion; and make-up cosmetics such as nail polish, mascara, or foundation, and above all, it can be suitably used as hair cosmetics.
The said amphoteric urethane resin having carboxyl group(s) and tertiary amino group(s) in one molecule may be prepared by making a prepolymer having isocyanate group(s) by reacting the polyol compound (component A), polyisocyanate compound (component B), and compound C having active hydrogen(s) and carboxyl group(s) with each other in an excess of isocyanate group(s), and allowing the said prepolymer containing isocyanate group(s) to react with the compound (component D) having active hydrogen(s)and tertiary amino group(s). Alternatively, the said amphoteric urethane resin may be also prepared by making a prepolymer having isocyanate group(s) by reacting component C and component D in reverse order, namely by allowing the said compound A, compound B, and compound D to react with each other in an excess of isocyanate group(s), and then, allowing the prepolymer containing the

isocyanate group(s) to react with the said specific compound C. Such processes make it possible to produce amphoteric urethane resin more easily and safely than conventional processes. In the production process as set forth, if the reaction between the compounds A and B is made concurrently with reaction between the specific compounds C and D, the carboxyl group(s) in compound C and the tertiary amino group(s) in compound D will form salts earlier, and it follows therefore that the resultant is insoluble in the reaction system, so that it will not react with isocyanate compounds even if it has an OH group, whereby the intended amphoteric urethane resin can not be produced.

Referring to the said polyol compound (component A), it can be any element that can be utilized in the production of common type polyurethane. General examples are polyesther polyol, polyether polyol, polycarbonate polyol, polybutadiene polyol, polyisoprene polyol, polyolefin polyol, ester polyacrylate polyol, etc. These components may be used separately or in conjunction with more than two kinds thereof. Above all, polyester polyol and polyether polyol may be adequately used.

Examples of the said polyester polyol are compounds originated from condensation polymerization of at least one kind of dicarboxylic acid such as succinic acid, glutaric acid, adipic acid, sebacic acid, azelaic acid, maleic acid, fumaric acid, phthalic acid, terephthalic acid; and at least one kind of polyhydric alcohol such as ethylene glycol, propylene glycol, 1,4-butanediol, 1,3-butanediol, 1,6-hexane diol, neopentyl glycol, 1,8-octane diol, 1,10-decane diol, diethylene glycol, spiro glycol, trimetylolpropane; or those obtained by the ring opening polymerization of lactones.

Referring to said polyether polyol, in addition to the polyhydric alcohol which is used in the synthesis of said polyester polyol, a compound resulted from ring opening polymerization of phenols such as bisphenol A, or primary amines or secondary amines with cyclic ether such as ethylene oxide, propylene oxide, oxetane, tetrahydrofuran may be employed, and a compound resulted from ring opening polymerization of polyoxyethylenepolyol, polyoxypropylenepolyol, polyoxytetramethylenepolyol, and bisphenol A with at least one of propyleneoxide and ethyleneoxide, etc. (in the case of copolymer, any of block copolymer and random copolymer may be available) can be cited by way of example.

Referring to polyisocyanate compound (component B), no specific element will be defined; organic diisocyanate compounds such as aliphatic di-isocyanate compound, cycloaliphatic diisocyanate compound, perfume di-isocyanate compound can be cited by way of example, and these compounds can be used separately or in conjunction with more than two kinds thereof.

Examples of the said aliphatic diisocyanate compounds are ethylenedi-isocyanate,2,2,4-trimethylhexamethylene diisocyanate, and 1,6-hexamethylene diisocyanate, etc. Example of the said cycloaliphatic diisocyanate compounds are hydrogenated 4,4-diphenylmethane diisocyanate, 1,4-cyclohexane diisocyanate, methylcyclohexylene diisocyanate, isophorone diisocyanate, and norbomane diisocyanate, etc. Examples of the said perfume diisocyanate compounds are 4,4-diphenylmethane di-isocyanate, xylene diisocyanate, toluene di-isocyanate, and naphthalene diisocyanate, etc. Of all these compounds, 1,6-hexamethylene diisocyanate, isophophorone diisocyanate, norbomane diisocyanate, etc. are preferable in that they are excellent in resistance to light and inexpensive.

Referring to the said compound (component C) having active hydrogen(s) and carboxyl group(s), any compound which includes at least one active hydrogen and at least one carboxyl group in its molecule suffices for the occasion. So, no specific compound will be defined. Examples are dimethylol propionic acid (DMPA), dimethylol butanoic acid, polycaprolactonediol containing carboxyl group(s), etc. They can be used separately or in conjunction with more than two kinds thereof.

Referring to the said compound (component D) having active hydrogen(s) and tertiary amino group(s), any compound which includes at least one active hydrogen and at least one tertiary amino group suffices for the occasion. So, no specific compound will be defined. Examples are N-alkyldialkanolamine compounds such as N-methyldiethanolamine, N-butyldiethanolamine, and dimethylaminoethanol, etc. They may be used separately or in conjunction with more than two kinds thereof.

When prepolymer containing isocyanate group(s)is produced using said components, chain extenders or molecular weight inhibitors can be used for regulation of characteristic features of amphoteric urethane resin as end product.

Referring to the said chain extender, no specific article will be defined. Examples of the article are low-molecular polyol and amines , etc. Examples of the said low-molecular polyol are glycols such as ethylene glycol, propylene glycol, 1,4-butanediol, diethylene glycol, 1,6 hexanediol, spiroglycol, cyclohexyl dimethanol, hydrogenated bisphenol A, neopenthylglycol, bis (-h ydroxyethoxy) benzene, and xylylene glycol; and triol such as trimethylolpropane, and glycerine , etc.. Examples of said amines are ethylene diamine, propylene diamine, piperazine, hydragine, isophorodiamine, methylene (bis-o-chloraniline), and polypropylene glycol containing amino groups at both ends.

Examples of the said molecular weight inhibitor are polypropylene glycol containing an amino group at one end and the like.

Solvent may be used in producing the said amphoteric urethane resin, as needed. For example, it is preferable to utilize organic solvent which can dissolve both raw materials and polyurethane to be prepared. Examples of the said organic solvent are amides such as N-methylpyrrolidone, dimethylformamide, and dimethylacetamide; ketones such as acetone and methyl ethyl ketone; esters such as ethyl acetate; and cellosolve acetate, and cellosolve ether, etc.

In the production of the said amphoteric urethane resin, the said resin can be provided with dispersibility in water by neutralizing the carboxyl group(s) or tertiary amino group(s) incorporated into the molecule by means of neutralizers, etc. Examples of neutralizers for use in the said carboxyl group are triethylamine, trimethylamine, 2-amino-2-methyl-1-propanol, triethanolamine, potassium hydroxide, and sodium hydroxide, etc. Examples of neutralizers for the said tertiary amino group are acetic acid, hydrochloric acid, sulfuric acid, nitric acid, and dimethyl sulfate, etc.

Polymerizing catalysts known in the field of polyurethane may be used in the production of the said amphoteric urethane resin. Examples of the said catalyst that can be utilized are tertiary amine catalyst and organic metal catalyst, etc. Examples of the said tertiary amine catalyst are [2,2,2] diazabicyclooctane (DABCO), tetramethylenediamine, N-methylmorpholine, and diazacycloundecene (DBU), etc. Examples of the said organic metal catalyst are dibutyltin (tin) dilaurate, etc.

Referring to the said amphoteric urethane resin, it is preferable to use resins of the type having structural unit (s) which is/are derived from ethylene oxide (EO), in terms of hair washability.

Examples of the structural unit which is derived from the said EO are the EO unit as shown in the following general formula (I), and propylene oxide (afterwards referred to as PO) unit as shown in the following formula (II), and the EO unit is more preferable.

$$-\!\!\left(CH_2\!-\!CH_2\!-\!O\right)_n\!\!- \qquad \ldots (I)$$

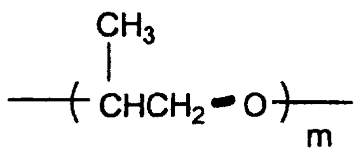

... (II)

The said amphoteric urethane resin may have both EO and PO units. The ratio of the said EO unit to PO unit is preferably within the range of 10/0 to 2/8, and more preferably 10/0 to 4/6.

The repeated number n of the EO unit in the said general formula (I) is set preferably within the range of 3 to 300, and more preferably 20 to 120. That is because if n is under 3, the EO unit(s) introduced into the amphoteric urethane resin is too little, so that the amphoteric urethane resin is not given sufficient hydrophilic nature, whereby there is a risk of the said resin obtaining insufficient hair washability. Conversely, if n exceeds 300, the hydrophilic nature of the amphoteric urethane resin is too high, which may affect the moisture vapour resistance. Also, the repeated number m of the PO unit in the said general formula (II) is set preferably within the range of 3 to 300, and more preferably 20 to 120. If the said amphoteric urethane resin has both the said EO unit and PO unit, n + m preferably ranges from 3 to 300, and more preferably from 20 to 120.

The amphoteric urethane resin having the structural unit(s) which is/are derived from the said ethyleneoxide (EO) is produced by making a prepolymer having isocyanate group(s), for instance, by allowing the polyol compound (component A), the polyisocyanate compound (component B), the polyethylene oxide derivative having active hydrogen (s), and a compound (component C) having active hydrogen(s) and carboxyl group(s) to react with each other in an excess of isocyanate group(s) and then, allowing the said prepolymer containing isocyanate group(s) to react with a compound (component D) having active hydrogen(s) and tertiary amino group(s). Alternatively, this production process can be effective even by conducting the reaction with component C and the reaction with component D in reverse order. In this case, the same compounds as aforementioned may be used as the said components A to D.

Referring to the polyethyleneoxide derivative having active hydrogen(s), any element that can introduce a structural unit that is derived from ethyleneoxide (EO) into the structure of the said amphoteric urethane resin may be utilized. So, no specific element will be defined. Examples are polyoxyethylene glycol(PEG),and polyoxyethylene polyoxypropylene glycol (EOPO block copolymer), etc., and more preferably, preoxyethylene glycol. The said polyethylene oxide derivative of any of the following types can be utilized; both-end OH group introduction type, both-end NH2 group introduction type, one-end OH group introduction type, and one-end NH2 introduction type. Amphoteric urethane resin having EO unit(s) in the main chain can be obtained by the use of the said both-end OH group introduction type or both-end NH2 group introduction type. Amphoteric urethane resin having EO unit(s) in the side chain(s) or end(s) can be obtained by the use of one-end OH group introduction type or one-side NH2 group introduction type.

The molecular weight of the said specific polyethylene oxide derivative is preferably 200 to 20000, and more

preferably 1000 to 10000.

It is preferable to use amphoteric urethane resin having polysiloxane bond(s) in the structure thereof for much greater improvement of the feel.

Referring to the said polysiloxane bond(s), the repeated number n of siloxane bond (Si-O) is preferably within the range of 5 to 300, and more preferably 20 to 150. This is because if n is less than 5, the proportion of siloxane bonds present in an amphoteric urethane resin being obtained may be too low, whereby sufficient effects which may be essentially acquired by the introduction of polysiloxane bond(s)s are hardly obtainable in touch, combing ability, etc. and conversely, with n exceeding 300, the compatibility with other materials will deteriorate due to a high level of its hydrophobic nature, thus leading to poor reaction. Moreover, there is the possibility that the hydrophobic nature of a resultant polymer is at so a high level that it may hinder the adhesion to hair.

Amphoteric urethane resin having the said polysiloxane bond(s) may be produced by making a prepolymer having isocyanate group(s), for example, by causing polylol compound (component A), polyisocyante compound (component B), polysiloxane compound having active hydrogen(s), and a compound having active hydrogen(s) and carboxyl group(s) (component C) to react with each other in an excess of isocyanate group(s), and then, reacting the resultant prepolymer containing isocyanate group(s) with a compound (compound D)having active hydrogen(s) and tertiary amino group(s). Alternatively, this production process can be effective even by conducting the reaction with component C and the reaction with component D in reverse order. In this case, the same compounds as aforementioned may be used as said components A to D.

Referring to the polysiloxane compound having active hydrogen(s) that is used together with the said components A to D, any compound that can introduce the polysiloxane bond(s) into the structure of the said amphoteric urethane resin suffices for the occasion. So, no specific compound will be defined. Examples of said compound are polydialkylsiloxanediol, polydialkylsiloxanemonool, polydialkylsiloxaneamine, and polydialkylsiloxanemonoamine, etc. They may be used separately or in conjunction with more than two kinds thereof. For the alkyl group(s) which bond to Si of siloxane bond(s) of the said polydialkylsiloxanediol, etc., the number of carbons is preferably 1 to 10, and more preferably 1 to 5. It does not matter if the aforementioned polysiloxane compound incorporates the alkyl group(s) which bond to Si of siloxane bond(s) and have a different number of carbons intermingled with each other. Specifically, examples of the said polydialkylsiloxanediol are polydimethylsiloxanediol, polymethylsiloxanediol, etc. Examples of the said polydialkylsiloxanemonool are polydimethylsiloxanemonool, polymethylsiloxanemonool, etc. Examples of the said polydialkylsiloxanediamine are polydimethylsiloxanedimine, polymethylsiloxandediamine, etc. Examples of the said polydialkylsiloxanemonoamine are polydimethylsiloxanemonoamine, polymethylethylsiloxanemonoamine, etc.

Examples of the said polysiloxane compounds are those of both-end OH group introduction type, both-end NH2 group introduction type, one-end OH group introduction type, and one-end NH2 group introduction type, etc. The use of said compound of both-end OH group introduction type or both-end NH2 group introduction type may bring about amphoteric urethane resin having polysiloxane bond(s) in the main chain, and the use of the compound of one-end OH group introduction type or one-end NH2 group introduction type may bring about amphoteric urethane resin having polysiloxane bond(s) in the side chain(s)or at end(s).

In the cosmetics in accordance with the present invention, it is preferable to use amphoteric urethane resin as aqueous liquid. With the present invention, the aqueous liquid means the water dispersion in which amphoteric urethane resin is dispersed as well as the water solution in which amphoteric urethane resin is dissolved completely.

It is possible to make the water dispersion of said amphoteric urethane resin cross linkable by addition of a crosslinking agent such as a silane coupling agent. There is no restriction on adding various additives in order to impart storage stability to the said water dispersion. Examples of the additives are protective colloid agent, anti-bacterial agent, mildewproofing agent, etc.

Referring to water-soluble resin which may be used together with the said amphoteric urethane resin, any of resins that can be utilized as cosmetics suffices for this occasion. So, no specific resin will be defined. Every type of resin, i.e. nonionic, anionic, cationic, or amphoteric type can be employed. They may be used separately or in conjunction with more than two kinds thereof.

Examples of said nonionic resins are polyvinyl alcohol, polyvinyl pyrrolidone [(Luviskol K-12,17,30,60,80,90 made by BASF Corporation), (PVP K-15,30,60,90,120 made by International Specialty Products)], vinylpyrrolidone-vinyl acetate copolymer [(Luviskol VA28,37,55,64,73 made by BASF Corporation), (PVP/VA-735,535,335,235,S-630 made by International Specialty Products), (PVA-6450 made by OSAKA ORGANIC CHEMICAL IND.LTD), vinylpyrrolidone-vinyl acetate-vinyl propionate copolymer (Luviskol VA343 made by BASF Corporation), vinyl pyrrolidone-vinyl acetateacrylamino acrylate copolymer, vinyl acetate/N-vinyl-5-methyl-2-oxazoline copolymer (Dowlex made by Dow Chemical Co.,Ltd), hydroxyethyl cellulose, hydroxypropyl cellulose, hydroxypropyl methyl cellulose, methyl cellulose, carboxymethyl cellulose, carboxyvinyl polymer, polyvinylformamide, and polyvinylacetamide , etc.

Examples of said anionic resins are vinyl acetate-crotonate copolymer [(Resin 28-1310 made by National Starch and Chemical Co.), (Luviset CA made by BASF Corporation), crotonic acid-vinyl acetate-vinyl neodecanate copolymer (Resin 28-2930 made by National Starch and Chemical Co.), crotonic acid-vinyl acetate-vinyl propionate

copolymer (Luviset CAP made by International Specialty Products), vinyl acetate-monobutyl maleate-isoboronyl acrylate copolymer (ADVANTAGE-CP made by International Specialty Products), N-octyl acrylamide/alkyl acrylates copolymer [(AMPHOMER HC made by National Starch and Chemical Co.), (Ultrahold 8, Ultrahold Strong made by BASF Corporation), vinyl pyrrolidone-acrylate-(meth) acrylic acid copolymer (Luviflex VBM35 , made by BASF Corporation), acrylic resin alkanol amine liquid as (meth) acrylic acid-alkyl(meth)acrylate copolymer [(Pluscise series made by GOO Chemical Co., Ltd.), (Aniset KB-1000, KB-100H, NF-1000, HS-3000, AQ-2500 made by OSAKA ORGANIC CHEMICAL IND.LTD.), (Diahold made by Mitsubishi Chemical Corporation), alkyl acrylate-alkyl methacrylate-diaceton acrylamide-methacrylic acid copolymer (Pluscise L-53 made by GOO Chemical Co., Ltd.), methyl vinyl ether-maleic anhydride alkyl halfester copolymer [(Gantrez ES-225, ES-425, SP-215 made by International Specialty Products), (Anieres BEM-42S, VEM-22S made by OSAKA ORGANIC CHEMICAL IND.LTD.).

Examples of said cationic resins are (lower nitrogen) hydroxyethyl cellulose dimethyldiallylammonium chloride (Celquat L-200, H-100 made by National Starch and Chemical Co.), O- [2-hydroxy-3-(trimethylammonio) propyl] hydroxyethyl cellulose chloride (Celquat SC240C, SC230M made by National Starch and Chemical Co.), vinyl pyrrolidone-quatemary dimethylaminoethyl methacrylate copolymer [(Gafquat 734, 755, 755N made by International Specialty Products), (H.C. Polymer 1S, 1N, 1NS, 1NP, 2, 2L, 3A, 5 made by OSAKA ORGANIC CHEMICAL IND.LTD.)], vinylimidazolium trichloride-vinylpyrrolidone copolymer (Luviquat FC370, FC550, FC905, HM552, MonoCP made by BASF Corporation), vinylpyrrolidone-dimethylaminoethyl methacrylate copolymer (Luviflex made by BASF Corporation), vinylpyrrolidone-alkylaminoacrylatevinylcaprolactam copolymer (copolymer 845, 937, 958 made by International Specialty Products), vinylpyrrolidonemethacrylamidepropyl trimethylammonium chloride copolymer (Gafquat HS-100 made by International Specialty Products), alkylacrylamide-acrylate-alkylaminoalkylamide-polyethylene glycol methacrylate copolymer, polydimethylethylenepiperidinium chloride liquid (Marquat 100 made by CALGON Corporation), dimethyldiallylammonium chloride-acrylamide copolymer (Marquat 2200, 550 made by CALGON Corporation), and cationic guagum, etc.

Examples of the said amphoteric resin are octylamide acrylate-hydroxypropylacrylate-butylaminoethyl methacrylate copolymer (AMPHOMER 28-4910, LV-71 made by National Starch and Chemical Co.), N-methacryloyloxyethyl-N, N-dimethylammonium-N-methylcarboxybetaine/alkyl methacrylate copolymer [(Yukaformer R205, R205S, SM, 301, 510, AM75, AM75S made by Mitsubishi Chemical Corporation), (RAM Resin-1000, 2000 made by OSAKA ORGANIC CHEMICAL IND.LTD.), acrylic acid-methacrylic acid-acrylic acid 2-hydroxypropyl-N, N-dimethylaminoethyl methacrylate-ethyl methacrylate-diacetonacrylamide-vinylpyrrolidone copolymer and its alkanolamine liquid (AP Polymer 560 made by OSAKA ORGANIC CHEMICAL IND.LTD.), etc.

The average molecular weight of these water-soluble resins is preferably within the range of 100 to 10,000,000, and more preferably from 10,000 to 5,000,000.

The blending proportion of the said amphoteric urethane resin to water-soluble resin in weight is preferably between 0.1/100 and 100/0.1, and more preferably 50/1 and 1/50.

In addition to the amphoteric urethane resins and water-soluble resins, the cosmetics in accordance with the present invention may contain other ingredients which are commonly used in normal cosmetics, such as pigment, colouring matter, colouring material, perfume, surfactant, humectant, conservation agent, preservative, disinfectant, antioxidant. lubricant, thickening agent, ultraviolet absorber, silicone polymer derivative, etc.

The cosmetics of the present invention may be produced in the following manner:

Production of hair cosmetics (foam hair fixative)

Aqueous liquid of the amphoteric urethane resins thus obtained as mentioned above is blended with water-soluble resin; various kinds of surfactants such as-polyoxy-ethylene alkyl-ether, coconut oil aliphatic acid diethanolamide, etc.; ethanol; de-ionized water, etc. in a predetermined proportion, and the resultant compositions are mixed until they are made homogenous to produce a component X. Then, the addition of a component Y consisting of propellant (LPG), etc. to the said component X makes the intended foam hair fixative.

Production of hair cosmetics (aerosol spray-type hair fixative)

Aqueous liquid of the amphoteric urethane resins thus obtained as above-mentioned is combined with water-soluble resin, de-ionized water, dioctylsodium sulfosuccinate, ethanol, etc., in a predetermined proportion, and the resultant compositions are mixed until they are made homogenous to produce a component X. Then, the addition of a component Y consisting of propellant (LPG), etc., to the said component X makes the intended aerosol spray-type hair fixative.

Production of hair cosmetics (gel-like hair fixative)

First, thickening agent, triethanolamine, ethanol, and de-ionized water, etc. are combined with each other in a predetermined proportion, and the resultant compositions are mixed until viscous gel is formed into a component X. Then, aqueous liquid of the said amphoteric urethane resin is combined with water-soluble resin, ethanol, de-ionized water, etc. in a predetermined proportion to obtain a component Y Then, this component Y is added to said component X, and the resultant compositions are mixed until they are made homogeous to produce the intended gel-like hair fixative.

Production of hair cosmetics (pumping spray-type hair fixative)

Aqueous water of the said amphoteric urethane resin is combined with water-soluble resin, dioctylsodium sulfosuccinate, ethanol, de-ionized water, etc. in a predetermined proportion, and the resultant compositions are mixed until they are made homogenous to produce the intended pumping spray-type hair fixative.

Skin care cosmetics such as shaving cream, skin care lotion, , sunscreen lotion, etc.; and, make-up cosmetics such as nail polish, mascara and foundation, etc. can be produced in accordance with the general production processes for these cosmetics.

Next, the Examples will be described with reference to Comparative Examples.

Prior to the description of the Examples and Comparative Examples, the following materials have been prepared.

Amphoteric urethane resin (a)

100g of isophorone diisocyanate (IPDI), 60g of polypropyrene glycol (PPG, molecular weight: 1000), 5g of cyclohexyldimethanol (CHDM), and 38g of dimethylol butanoic acid(DMBA) were put into a glass flask with four openings and equipped with an agitator, a thermometer, a nitrogen duct, and a reflux condenser, then, 60g of ethyl acetate was added as a solvent, and the materials were heated at a temperature of 80°C in the oil bath and allowed to react for four hours. Then, 2g of N-methyldiethanol amine and 30g of ethyl acetate were added, and the mixture was held for three hours for reaction purpose. Moreover, 30g of polypropyrene glycol having one amino group at one end thereof (Jeffamine M1000 made by HUNTSMAN CORPORATION) and 50g of ethyl acetate were added to the mixture, and the mixture was allowed to react for one hour, thereby obtaining a solution of polyurethane prepolymer with the residual NCO groups remaining therein. This polyurethane prepolymer with the residual NCO groups was dispersed into 750g of water containing 16g of potassium hydroxide, and subjected to chain extension at 50°C for three hours for polymerization. Ethyl acetate was recovered from water dispersion thus obtained under a low pressure to obtain aqueous matters of amphoteric urethane resin without substantial amounts of solvent.

Amphoteric urethane resin (b)

100g of isophorone diisocyanate (IPDI), 60g of propyrene glycol (PPG, molecular weight:1000), 5g of cyclohexyl dimethanol (CHDM), 20g of polyoxyethylene glycol (PEG, molecular weight:1000), and 36g of dimethylol butanoic acid (DMBA) were put into the glass flask with four openings and equipped with an agitator, a thermometer, a nitrogen duct and a reflux condenser, then, 60g of ethyl acetate as a solvent were added and the mixture was heated at a temperature 80 C in the oil bath to react for four hours. Then, 2g of N-methyldiethanol amine and 30g of ethyl acetate were added, and the mixture was held for three hours for reaction purpose. Moreover, 30g of polypropyrene glycol having one amino group at one end thereof (Jeffamine M1000 made by HUNTSMAN CORPORATION) and 50g of ethyl acetate were added to the mixture, and the mixture was allowed to react for one hour, thereby obtaining a solution of polyurethane prepolymer with residual NCO groups therein. This polyurethane polymer with residual NCO groups therein was dispersed in 750g of water containing 15g of potassium hydroxide and subjected to chain extension at a temperature of 50C for three hours for polymerization. , Ethyl acetate was recovered from the resulting water dispersion under a low pressure to obtain aqueous matters of amphoteric urethane resin without substantial amounts of solvent but including ethyleneoxide chains in its structure.

Amphoteric urethane resin (c)

100g of isophorone diisocyanate (IPDI) and 3g of polydimethyl siloxanediol having two OH groups at one end thereof (molecular weight: 1000), were put into the glass flask with four openings and equipped with an agitator, a thermometer, a nitrogen duct and a reflux condenser and heated at a temperature of 80C i n the oil bath to react for two hours. Then, 55g of polypropylene glycol (PPG, molecular weight: 1000), 10g of hydrogenated bisphenol A, and

36g of dimethylol butanoic acid (DMBA) were added together with 60g of ethyl acetate as a solvent, and the mixture was heated at 80C in the oil bath to react for four hours. Then, 2g of N-methyldiethanol amine and 30g of ethyl acetate were added, and the mixture was allowed to react for another three hours. Moreover, 30g of polypropylene glycol having one amino group at one end thereof (Jeffamine M1000 made by HUNTSMAN CORPORATION) and 50g of ethyl acetate were added to the mixture, and the mixture was allowed to react for another one hour, thereby obtaining a solution of polyurethane prepolymer with residual NCO groups therein. This polyurethane prepolymer with residual NCO groups therein was dispersed in 750 g water containing 15g of potassium hydroxide, and subjected to chain extension at a temperature of 50C for three hours for polymerization. Ethyl acetate was recovered from the resulting water dispersion under a low pressure to obtain aqueous matters of amphoteric urethane resin without substantial amounts of solvent but incorporating dimethyl siloxane chains in its structure.

Water-soluble resin (1) (nonionic)

Polyvinyl formamide.

Water-soluble resin (2) (nonionic)

Polyvinyl pyrrolidone (Luviskol K-90 made by BASF Corporation).

Water-soluble resin (3) (anionic)

Crotonic acid-vinyl acetate-vinyl neodecanoic acid copolymer (Resin 28-2930 made by National Starch and Chemical Co.)

Water-soluble resin (4) (anionic)

Acrylic resin alkanolamine liquid (Pluscise L-9909B made by GOO Chemical Co., Ltd.)

Water-soluble resin (5) (cationic)

Hydroxy ethylcellulose-dimethyl diallyl ammoniumchloride (Celquat L-200 made by National Starch and Chemical Co.)

Water-soluble resin (6) (cationic)

Vinyl pyrrolidone-N, N-dimethyl aminoethyl methacrylic acid copolymer diethylsulfate (Gafquat 755N made by International Specialty Products)

Water-soluble resin (7) (amphoteric)

Octylamide acrylate-hydroxypropyl acrylate-butylamino ethyl methacrylate copolymer (AMPHOMER 28-4910 made by National Starch and Chemical Co.)

Water-soluble resin (8) (amphoteric)

N-methacryl oyloxyethyl-N, N-dimethylammonium-N-methyl carboxybetaine-alkyl methacrylate copolymer (Yukaformer SM made by Mitsubishi Chemical Corporation)

Polyoxyethylene stearyl ether

NIKKOL BS-20 made by NIKKO CHEMICALS CO., LTD.

Coconut oil aliphatic acid diethanolamide

Amicor CDE-1 made by MIYOSHI OIL & FAT CO., LTD.

Dioctyl sodium sulfosuccinate

Monawet MO-70E made by MONA INDUSTRIES INC.

Thickening agent

Alkyl acrylate-polyoxy ethylene stearyl ether itaconate copolymer (Structure 2001 made by National Starch and Chemical Co.)

Hair cosmetics (foam hair fixative)

Examples 1 a to 24a. Comparative Examples 1a to 11a

The X component was obtained by blending each material of the X components shown in the following Table 1 to 5 a t the ratios shown therein and mixing until homogeneous. Subsequently, the Y component was added to the X component at the ratios shown in the said Tables to make the foam hair fixative. The proportion of amphoteric urethane resin to water-soluble resin designates dry weight (this applies to the Examples and Comparative Examples.)

The properties of foam hair fixatives thus obtained in the Examples and Comparative Examples were evaluated in accordance with the following standards. The results are concurrently represented in the following Tables 1 to 5.

Touch

10 panelists conducted organoleptic tests on a sample of black virgin hair (length:25cm, weight:5.0g) which had been coated with 0.8g of foam hair fixative and dried at normal room temperature for evaluation of touch as should inherently be endowed with as hair cosmetics. The evaluation standard is as follows.

: 9～10 persons who felt the sample of hair to be very soft to the touch.
: 6～8 persons who felt the sample of hair to be very soft to the touch.
: 2～5 persons who felt the sample of hair to be very soft to the touch.
×: 0～1 person who felt the sample of hair to be very soft to the touch.

Durability

Five curls, each consisting of black virgin hair (length:15cm, weight:3g) which had been coated with 0.6g of foam hair fixative, were prepared for each test piece, and held dried at a temperature of 50°C throughout the night. Then, the dried samples of hair were hung on a board marked with a graded scale in the thermo-hygrostat where the temperature was maintained at 35°C and the moisture at 90%RH. The initial length of curl (a) and the length of curl (b) after five hours were measured to find the curl retentions in accordance with the following expression, wherein L is the length of a sample of hair which has been fully stretched.

$$\text{Curl retention (\%) } \{(L\ b)(L\ a)\} \times 100$$

It is shown that as the values of the said curl retention approach 100%, the maintenance of curls is stronger and the durability more excellent. The evaluation standard is as follows.

90% or over
70% to under 90%
50% to under 70%
× Under 50%

Hair washability

A well-dressed sample of hair was prepared using black virgin hair (length:15cm, weigth:3g) which had been coated with 0.6g of the foam hair fixative and dried at room temperature. Then, the said sample of hair was slightly loosen using warm water at 40C for 30 seconds, then 0.4g of 10% shampoo solvent was applied to the sample of hair which was then washed for 30 seconds. Then, it was rinsed with warm water at 40C again to remove the shampoo solvent, and dried sufficiently at 50C . Referring to the sample of hair thus arranged, 10 panelists conducted organoleptic

tests for hair-setting ability and evaluated its hair washability which the foam hair fixative should inherently be endowed with as hair cosmetics. The evaluation standard is as follows.

: 9~10 persons who felt the sample of hair after drying to be nil in setting ability but very excellent in hair washability.
: 6~8 persons who felt the sample of hair after drying to be nil in setting ability but very excellent in hair washability.
: 2~5 persons who felt the sample of hair after drying to be nil in setting ability but very excellent in hair washability.
×: 0~1 person who felt the sample of hair after drying to be nil in setting ability but very excellent in hair washability.

Feel

For a sample of black virgin hair (length:25cm, weight:5.0g) which had been coated with 0.8g of the foam hair fixative and dried at the room temperature, 10 panelists conducted organoleptic tests and evaluated the feel which the foam hair fixative should be inherently endowed with as hair cosmetics. The evaluation standard is as follows.

: 9~10 persons who felt the sample of hair after drying to have a very silky feel.
: 6~8 persons who felt the sample of hair after drying to have a very silky feel.
: 2~5 persons who felt the sample of hair after drying to have a very silky feel.
×: 0~1 person who felt the sample of hair after drying to have a very silky feel.

Table 1

(Foam hair fixative) (Part)

| | | | Example | | | | | | | |
|---|---|---|---|---|---|---|---|---|---|---|
| | | | 1a | 2a | 3a | 4a | 5a | 6a | 7a | 8a |
| Component X | Amphoteric urethane resin | | 1.5 | 1.5 | 1.5 | 1.5 | 1.5 | 1.5 | 1.5 | 1.5 |
| | (Kind) | | (a) | (a) | (a) | (a) | (a) | (a) | (a) | (a) |
| | Water-soluble resin | | 1.5 | 1.5 | 1.5 | 1.5 | 1.5 | 1.5 | 1.5 | 1.5 |
| | (Kind) | | (1) | (2) | (3) | (4) | (5) | (6) | (7) | (8) |
| | De-ionized water | | 77.7 | 77.7 | 77.7 | 77.7 | 77.7 | 77.7 | 77.7 | 77.7 |
| | 2-amino-2-methyl-1-propanol | | - | - | Proper quantity | - | - | - | Proper quantity | - |
| | Polyoxyethylene stearyl ether | | 0.5 | 0.5 | 0.5 | 0.5 | 0.5 | 0.5 | 0.5 | 0.5 |
| | Ethanol | | 10.0 | 10.0 | 10.0 | 10.0 | 10.0 | 10.0 | 10.0 | 10.0 |
| | Coconut oil aliphatic acid diethanolamide | | 0.8 | 0.8 | 0.8 | 0.8 | 0.8 | 0.8 | 0.8 | 0.8 |
| Component Y | | Propellant (LPG) | 8.0 | 8.0 | 8.0 | 8.0 | 8.0 | 8.0 | 8.0 | 8.0 |
| Touch | | | | | | | | | | |
| Durability | | | | | | | | | | |
| Hair washability | | | | | | | | | | |
| Feel | | | | | | | | | | |

**Table 2**

(Foam Hair Fixative) (Part)

| | | Example | | | | | | | |
|---|---|---|---|---|---|---|---|---|---|
| | | 9a | 10a | 11a | 12a | 13a | 14a | 15a | 16a |
| Component X | Amphoteric urethane resin | 1.5 | 1.5 | 1.5 | 1.5 | 1.5 | 1.5 | 1.5 | 1.5 |
| | (Kind) | (b) | (b) | (b) | (b) | (b) | (b) | (b) | (b) |
| | Water-soluble resin | 1.5 | 1.5 | 1.5 | 1.5 | 1.5 | 1.5 | 1.5 | 1.5 |
| | (Kind) | (1) | (2) | (3) | (4) | (5) | (6) | (7) | (8) |
| | De-ionized water | 77.7 | 77.7 | 77.7 | 77.7 | 77.7 | 77.7 | 77.7 | 77.7 |
| | 2-amino-2-methyl-1-propanol | - | - | Proper quantity | - | - | - | Proper quantity | - |
| | Polyoxyethylene stea-Ryl ether | 0.5 | 0.5 | 0.5 | 0.5 | 0.5 | 0.5 | 0.5 | 0.5 |
| | Ethanol | 10.0 | 10.0 | 10.0 | 10.0 | 10.0 | 10.0 | 10.0 | 10.0 |
| | Coconut oil aliphatic acid diethanolamide | 0.8 | 0.8 | 0.8 | 0.8 | 0.8 | 0.8 | 0.8 | 0.8 |
| Component Y | Propellant (LPG) | 8.0 | 8.0 | 8.0 | 8.0 | 8.0 | 8.0 | 8.0 | 8.0 |
| Touch | | | | | | | | | |
| Durability | | | | | | | | | |
| Hair washability | | | | | | | | | |
| Feel | | | | | | | | | |

Table 3

(Foam hair fixative) (Part)

| | | Example | | | | | | | |
|---|---|---|---|---|---|---|---|---|---|
| | | 17a | 18a | 19a | 20a | 21a | 22a | 23a | 24a |
| Component X | Amphoteric urethane Resin | 1.5 | 1.5 | 1.5 | 1.5 | 1.5 | 1.5 | 1.5 | 1.5 |
| | (Kind) | (c) | (c) | (c) | (c) | (c) | (c) | (c) | (c) |
| | Water-soluble resin | 1.5 | 1.5 | 1.5 | 1.5- | 1.5 | 1.5 | 1.5 | 1.5 |
| | (Kind) | (1) | (2) | (3) | (4) | (5) | (6) | (7) | (8) |
| | De-ionized water | 77.7 | 77.7 | 77.7 | 77.7 | 77.7 | 77.7 | 77.7 | 77.7 |
| | 2-amino-2-methyl-1-propanol | - | - | Proper quantity | - | - | - | Proper quantity | - |
| | Polyoxyethylene stearyl ether | 0.5 | 0.5 | 0.5 | 0.5 | 0.5 | 0.5 | 0.5 | 0.5 |
| | Ethanol | 10.0 | 10.0 | 10.0 | 10.0 | 10.0 | 10.0 | 10.0 | 10.0 |
| | Coconut oil aliphatic acid diethanolamide | 0.8 | 0.8 | 0.8 | 0.8 | 0.8 | 0.8 | 0.8 | 0.8 |
| Component Y | Propellant (LPG) | 8.0 | 8.0 | 8.0 | 8.0 | 8.0 | 8.0 | 8.0 | 8.0 |
| Touch | | | | | | | | | |
| Durability | | | | | | | | | |
| Hair washability | | | | | | | | | |
| Feel | | | | | | | | | |

Table 4

(Foam hair fixative) (Part)

| | | Comparative Example | | | | | | | |
|---|---|---|---|---|---|---|---|---|---|
| | | 1a | 2a | 3a | 4a | 5a | 6a | 7a | 8a |
| Component X | Amphoteric urethane resin | - | - | - | - | - | - | - | - |
| | (Kind) | - | - | - | - | - | - | - | - |
| | Water-soluble resin | 3.0 | 3.0 | 3.0 | 3.0 | 3.0 | 3.0 | 3.0 | 3.0 |
| | (Kind) | (1) | (2) | (3) | (4) | (5) | (6) | (7) | (8) |
| | De-ionized water | 77.7 | 77.7 | 77.7 | 77.7 | 77.7 | 77.7 | 77.7 | 77.7 |
| | 2-amino-2-methyl-1-propanol | - | - | Proper quantity | - | - | - | Proper quantity | - |
| | Polyoxyethylene stearyl ether | 0.5 | 0.5 | 0.5 | 0.5 | 0.5 | 0.5 | 0.5 | 0.5 |
| | Ethanol | 10.0 | 10.0 | 10.0 | 10.0 | 10.0 | 10.0 | 10.0 | 10.0 |
| | Coconut oil aliphatic acid diethanolamide | 0.8 | 0.8 | 0.8 | 0.8 | 0.8 | 0.8 | 0.8 | 0.8 |
| Component Y | Propellant (LPG) | 8.0 | 8.0 | 8.0 | 8.0 | 8.0 | 8.0 | 8.0 | 8.0 |
| Touch | | | | | | | | | |
| Durability | | | | | | | | | |
| Hair washability | | | | | | | | | |
| Feel | | | | | | | | | |

Table 5

(Foam Hair Fixative) (Part)

| | | Comparative Examples | | |
|---|---|---|---|---|
| | | 9a | 10a | 11a |
| Component X | Amphoteric urethane resin | 3.0 | 3.0 | 3.0 |
| | (Types) | (a) | (b) | (c) |
| | Water-soluble resin | - | - | - |
| | (Kind) | - | - | - |
| | Deionized water | 77.7 | 77.7 | 77.7 |
| | 2-amino-2-methyl-1-propanol | - | - | - |
| | Polyoxyethylene stearyl ether | 0.5 | 0.5 | 0.5 |
| | Ethanol | 10.0 | 10.0 | 10.0 |
| | Coconut oil fatty acid diethanolamide | 0.8 | 0.8 | 0.8 |
| Component Y | Propellant (LPG) | 8.0 | 8.0 | 8.0 |
| Touch | | | | |
| Durability | | | | |
| Hair washability | | | | |
| Feel | | | | |

It is clear from the above Table 1 to 5 that the Examples of the foam hair fixative have very good touch, good feel, and ensure excellent hair washability because of the amphoteric urethane resin content, and are high in curl retention and excellent in durability because of the water-soluble resin content.

Particularly, the Examples of the foam hair fixative, using anionic and amphoteric water-soluble resin, are extremely high in curl retention and excellent in durability. Also, the Examples of the foam hair fixative, using amphoteric urethane resin (b) structurally having ethylene oxide chain(s), ensure excellent hair washability. Furthermore, the Examples of the foam hair fixative, using amphoteric urethane resin (c) structurally having polysiloxane bond(s), have very good feel.

In contrast, it is clear that the Comparative Examples 1a to 8a of the foam hair fixative containing water-soluble resin are high in curl retention and excellent in durability and that the said examples containing no amphoteric urethane resin have very bad touch. Also, the Comparative Example 9a to 11a containing amphoteric urethane resin have very good touch and that the said examples containing no water-soluble resin are low in curl retention and poor in durability.

<u>Hair cosmetics (aerosol spray-type hair fixative)</u>

<u>Examples 1 b to 24b. Comparative Examples 1 b to 11 b</u>

The X component was obtained by blending each material of the X component shown in the following Table 6 to 10 at the ratios shown therein and mixing until becoming homogeneous. Subsequently, the Y component was added to the X component at the ratios shown in the said Tables to make the aerosol spray-type hair fixative.

The properties of the aerosol spray type hair fixatives thus obtained in the Examples and Comparative Examples were evaluated in accordance with the standard for the aforementioned cosmetics for hair. The results are concurrently represented in the following Tables 6 to 10.

Table 6

(Aerosol spray-type hair fixative) (Part)

| | | | Example | | | | | | | |
|---|---|---|---|---|---|---|---|---|---|---|
| | | | 1b | 2b | 3b | 4b | 5b | 6b | 7b | 8b |
| Component X | Amphoteric urethane Resin | | 1.5 | 1.5 | 1.5 | 1.5 | 1.5 | 1.5 | 1.5 | 1.5 |
| | (Kind) | | (a) | (a) | (a) | (a) | (a) | (a) | (a) | (a) |
| | Water-soluble resin | | 1.5 | 1.5 | 1.5 | 1.5 | 1.5 | 1.5 | 1.5 | 1.5 |
| | (Kind) | | (1) | (2) | (3) | (4) | (5) | (6) | (7) | (8) |
| | 2-amino-2-methyl-1-propanol | | - | - | Proper quantity | - | - | - | Proper quantity | - |
| | De-ionized water | | 7.0 | 7.0 | 7.0 | 7.0 | 7.0 | 7.0 | 7.0 | 7.0 |
| | Diocty sodium sulfosuccinate | | 0.3 | 0.3 | 0.3 | 0.3 | 0.3 | 0.3 | 0.3 | 0.3 |
| | Ethanol | | 49.7 | 49.7 | 49.7 | 49.7 | 49.7 | 49.7 | 49.7 | 49.7 |
| Component Y | | Propellant (LPG) | 40.0 | 40.0 | 40.0 | 40.0 | 40.0 | 40.0 | 40.0 | 40.0 |
| Touch | | | | | | | | | | |
| Durability | | | | | | | | | | |
| Hair washability | | | | | | | | | | |
| Feel | | | | | | | | | | |

Table 7

(Aerosol spray-type hair fixative) (Part)

| | | Examples | | | | | | | |
|---|---|---|---|---|---|---|---|---|---|
| | | 9b | 10b | 11b | 12b | 13b | 14b | 15b | 16b |
| Component X | Amphoteric urethane Resin | 1.5 | 1.5 | 1.5 | 1.5 | 1.5 | 1.5 | 1.5 | 1.5 |
| | (Kind) | (b) | (b) | (b) | (b) | (b) | (b) | (b) | (b) |
| | Water-soluble resin | 1.5 | 1.5 | 1.5 | 1.5 | 1.5 | 1.5 | 1.5 | 1.5 |
| | (Kind) | (1) | (2) | (3) | (4) | (5) | (6) | (7) | (8) |
| | 2-amino-2-methyl-1-propanol | - | - | Proper quantity | - | - | - | Proper quantity | - |
| | De-ionized water | 7.0 | 7.0 | 7.0 | 7.0 | 7.0 | 7.0 | 7.0 | 7.0 |
| | Diocty sodium sulfosuccinate | 0.3 | 0.3 | 0.3 | 0.3 | 0.3 | 0.3 | 0.3 | 0.3 |
| | Ethanol | 49.7 | 49.7 | 49.7 | 49.7 | 49.7 | 49.7 | 49.7 | 49.7 |
| Component Y | Propellant (LPG) | 40.0 | 40.0 | 40.0 | 40.0 | 40.0 | 40.0 | 40.0 | 40.0 |
| Touch | | | | | | | | | |
| Durability | | | | | | | | | |
| Hair washability | | | | | | | | | |
| Feel | | | | | | | | | |

Table 8

(Aerosol spray-type hair fixative) (Part)

| | | Examples | | | | | | | |
|---|---|---|---|---|---|---|---|---|---|
| | | 17b | 18b | 19b | 20b | 21b | 22b | 23b | 24b |
| Component X | Amphoteric urethane resin | 1.5 | 1.5 | 1.5 | 1.5 | 1.5 | 1.5 | 1.5 | 1.5 |
| | (Kind) | (c) | (c) | (c) | (c) | (c) | (c) | (c) | (c) |
| | Water-soluble resin | 1.5 | 1.5 | 1.5 | 1.5 | 1.5 | 1.5 | 1.5 | 1.5 |
| | (Kind) | (1) | (2) | (3) | (4) | (5) | (6) | (7) | (8) |
| | 2-amino-2-methyl-1-propanol | - | - | Proper quantity | - | - | - | Proper quantity | - |
| | De-ionized water | 7.0 | 7.0 | 7.0 | 7.0 | 7.0 | 7.0 | 7.0 | 7.0 |
| | Diocty sodium sulfosuccinate | 0.3 | 0.3 | 0.3 | 0.3 | 0.3 | 0.3 | 0.3 | 0.3 |
| | Ethanol | 49.7 | 49.7 | 49.7 | 49.7 | 49.7 | 49.7 | 49.7 | 49.7 |
| Component Y | Propellant (LPG) | 40.0 | 40.0 | 40.0 | 40.0 | 40.0 | 40.0 | 40.0 | 40.0 |
| Touch | | | | | | | | | |
| Durability | | | | | | | | | |
| Hair washability | | | | | | | | | |
| Feel | | | | | | | | | |

Table 9

(Aerosol spray-type hair fixative) (Part)

| | | Comparative Example | | | | | | | |
|---|---|---|---|---|---|---|---|---|---|
| | | 1b | 2b | 3b | 4b | 5b | 6b | 7b | 8b |
| Component X | Amphoteric urethane resin | - | - | - | - | - | - | - | - |
| | (Kind) | - | - | - | - | - | - | - | - |
| | Water-soluble resin | 3.0 | 3.0 | 3.0 | 3.0 | 3.0 | 3.0 | 3.0 | 3.0 |
| | (Kind) | (1) | (2) | (3) | (4) | (5) | (6) | (7) | (8) |
| | 2-amino-2-methyl-1-propanol | - | - | Proper quantity | - | - | - | Proper quantity | - |
| | De-ionized water | 7.0 | 7.0 | 7.0 | 7.0 | 7.0 | 7.0 | 7.0 | 7.0 |
| | Diocty sodium sulfosuccinate | 0.3 | 0.3 | 0.3 | 0.3 | 0.3 | 0.3 | 0.3 | 0.3 |
| | Ethanol | 49.7 | 49.7 | 49.7 | 49.7 | 49.7 | 49.7 | 49.7 | 49.7 |
| Component Y | Propellant (LPG) | 40.0 | 40.0 | 40.0 | 40.0 | 40.0 | 40.0 | 40.0 | 40.0 |
| Touch | | | | | | | | | |
| Durability | | | | | | | | | |
| Hair washability | | | | | | | | | |
| Feel | | | | | | | | | |

Table 10

(Aerosol spray-type hair fixative) (Part)

| | | Comparative Example | | |
|---|---|---|---|---|
| | | 9b | 10b | 11b |
| Component X | Amphoteric urethane resin | 3.0 | 3.0 | 3.0 |
| | (Kind) | (a) | (b) | (c) |
| | Water-soluble resin | - | - | - |
| | (Kind) | - | - | - |
| | 2-amino-2-methyl-1-propanol | - | - | - |
| | De-ionized water | 7.0 | 7.0 | 7.0 |
| | Diocty sodium sulfosuccinate | 0.3 | 0.3 | 0.3 |
| | Ethanol | 49.7 | 49.7 | 49.7 |
| Component Y | Propellant (LPG) | 40.0 | 40.0 | 40.0 |
| Touch | | | | |
| Durability | | | | |
| Hair washability | | | | |
| Feel | | | | |

It is clear from the above Table 6 to 10 that the Examples of the aerosol spray-type hair fixative have very good touch, good feel, and ensure excellent hair washability because of containing amphoteric urethane resin, and are high in curl retention and excellent in durability because of the water-soluble resin content.

In contrast, it is clear that the Comparative Examples 1 b to 8b of the aerosol spray-type hair fixative containing no amphoteric urethane resin have very bad touch, and that the Comparative Examples 9b to 11 b of the aerosol spray-type hair fixative containing no water-soluble resin are low in curl retention and poor in durability.

Hair cosmetics (gel-like hair fixative)

Examples 1c to 24c. Comparative Examples 1c to 11c

The X component was obtained by blending each material of the X component shown in the following Table 11 to 15 at the ratios shown therein and mixing until forming of viscous gel. Subsequently, the Y component obtained by blending each material at the ratios shown in the said Tables, was added to the above-mentioned X component and mixed until becoming homogeneous to make the gel type hair fixative.

The properties of the gel type hair fixatives thus obtained in the Examples and Comparative Examples were evaluated in accordance with the standard for the aforementioned cosmetics for hair. The results are concurrently represented in the following Tables 11 to 15.

Table 11

(Gel-like hair fixative) (Part)

| | | Examples | | | | | | | |
|---|---|---|---|---|---|---|---|---|---|
| | | 1c | 2c | 3c | 4c | 5c | 6c | 7c | 8c |
| Component X | Thickening agent | 1.5 | 1.5 | 1.5 | 1.5 | 1.5 | 1.5 | 1.5 | 1.5 |
| | Triethanolamine | 1.1 | 1.1 | 1.1 | 1.1 | 1.1 | 1.1 | 1.1 | 1.1 |
| | Ethanol | 5.0 | 5.0 | 5.0 | 5.0 | 5.0 | 5.0 | 5.0 | 5.0 |
| | De-ionized water | 50.0 | 50.0 | 50.0 | 50.0 | 50.0 | 50.0 | 50.0 | 50.0 |
| Component Y | Amphoteric urethane resin | 1.5 | 1.5 | 1.5 | 1.5 | 1.5 | 1.5 | 1.5 | 1.5 |
| | (Kind) | (a) | (a) | (a) | (a) | (a) | (a) | (a) | (a) |
| | Water-soluble resin | 1.5 | 1.5 | 1.5 | 1.5 | 1.5 | 1.5 | 1.5 | 1.5 |
| | (Kind) | (1) | (2) | (3) | (4) | (5) | (6) | (7) | (8) |
| | 2-amino-2-methyl-1-propanol | - | - | Proper quantity | - | - | - | Proper quantity | - |
| | Ethanol | 5.0 | 5.0 | 5.0 | 5.0 | 5.0 | 5.0 | 5.0 | 5.0 |
| | De-ionized water | 34.4 | 34.4 | 34.4 | 34.4 | 34.4 | 34.4 | 34.4 | 34.4 |
| Touch | | | | | | | | | |
| Durability | | | | | | | | | |
| Hair washability | | | | | | | | | |
| Feel | | | | | | | | | |

Table 12

(Gel-like hair fixative) (Part)

| | | Examples | | | | | | | |
|---|---|---|---|---|---|---|---|---|---|
| | | 9c | 10c | 11c | 12c | 13c | 14c | 15c | 16c |
| Component X | Thickening agent | 1.5 | 1.5 | 1.5 | 1.5 | 1.5 | 1.5 | 1.5 | 1.5 |
| | Triethanolamine | 1.1 | 1.1 | 1.1 | 1.1 | 1.1 | 1.1 | 1.1 | 1.1 |
| | Ethanol | 5.0 | 5.0 | 5.0 | 5.0 | 5.0 | 5.0 | 5.0 | 5.0 |
| | De-ionized water | 50.0 | 50.0 | 50.0 | 50.0 | 50.0 | 50.0 | 50.0 | 50.0 |
| Component Y | Amphoteric urethane resin | 1.5 | 1.5 | 1.5 | 1.5 | 1.5 | 1.5 | 1.5 | 1.5 |
| | (Kind) | (b) | (b) | (b) | (b) | (b) | (b) | (b) | (b) |
| | Water-soluble resin | 1.5 | 1.5 | 1.5 | 1.5 | 1.5 | 1.5 | 1.5 | 1.5 |
| | (Kind) | (1) | (2) | (3) | (4) | (5) | (6) | (7) | (8) |
| | 2-amino-2-methyl-1-propanol | - | - | Proper quantity | - | - | - | Proper quantity | - |
| | Ethanol | 5.0 | 5.0 | 5.0 | 5.0 | 5.0 | 5.0 | 5.0 | 5.0 |
| | De-ionized water | 34.4 | 34.4 | 34.4 | 34.4 | 34.4 | 34.4 | 34.4 | 34.4 |
| Touch | | | | | | | | | |
| Durability | | | | | | | | | |
| Hair washability | | | | | | | | | |
| Feel | | | | | | | | | |

Table 13

(Gel-like hair fixative) (Part)

| | | Examples | | | | | | | |
|---|---|---|---|---|---|---|---|---|---|
| | | 17c | 18c | 19c | 20c | 21c | 22c | 23c | 24c |
| Component X | Thickening agent | 1.5 | 1.5 | 1.5 | 1.5 | 1.5 | 1.5 | 1.5 | 1.5 |
| | Triethanolamine | 1.1 | 1.1 | 1.1 | 1.1 | 1.1 | 1.1 | 1.1 | 1.1 |
| | Ethanol | 5.0 | 5.0 | 5.0 | 5.0 | 5.0 | 5.0 | 5.0 | 5.0 |
| | De-ionized water | 50.0 | 50.0 | 50.0 | 50.0 | 50.0 | 50.0 | 50.0 | 50.0 |
| Component Y | Amphoteric urethane resin | 1.5 | 1.5 | 1.5 | 1.5 | 1.5 | 1.5 | 1.5 | 1.5 |
| | (Kind) | (c) | (c) | (c) | (c) | (c) | (c) | (c) | (c) |
| | Water-soluble resin | 1.5 | 1.5 | 1.5 | 1.5 | 1.5 | 1.5 | 1.5 | 1.5 |
| | (Kind) | (1) | (2) | (3) | (4) | (5) | (6) | (7) | (8) |
| | 2-amino-2-methyl-1-propanol | - | - | Proper quantity | - | - | - | Proper quantity | - |
| | Ethanol | 5.0 | 5.0 | 5.0 | 5.0 | 5.0 | 5.0 | 5.0 | 5.0 |
| | De-ionized water | 34.4 | 34.4 | 34.4 | 34.4 | 34.4 | 34.4 | 34.4 | 34.4 |
| Touch | | | | | | | | | |
| Durability | | | | | | | | | |
| Hair washability | | | | | | | | | |
| Feel | | | | | | | | | |

Table 14

(Gel-like hair fixative) (Part)

| | | Comparative Examples | | | | | | | |
|---|---|---|---|---|---|---|---|---|---|
| | | 1c | 2c | 3c | 4c | 5c | 6c | 7c | 8c |
| Component X | Thickening agent | 1.5 | 1.5 | 1.5 | 1.5 | 1.5 | 1.5 | 1.5 | 1.5 |
| | Triethanolamine | 1.1 | 1.1 | 1.1 | 1.1 | 1.1 | 1.1 | 1.1 | 1.1 |
| | Ethanol | 5.0 | 5.0 | 5.0 | 5.0 | 5.0 | 5.0 | 5.0 | 5.0 |
| | De-ionized water | 50.0 | 50.0 | 50.0 | 50.0 | 50.0 | 50.0 | 50.0 | 50.0 |
| Component Y | Amphoteric urethane resin | - | - | - | - | - | - | - | - |
| | (Kind) | - | - | - | - | - | - | - | - |
| | Water-soluble resin | 3.0 | 3.0 | 3.0 | 3.0 | 3.0 | 3.0 | 3.0 | 3.0 |
| | (Kind) | (1) | (2) | (3) | (4) | (5) | (6) | (7) | (8) |
| | 2-amino-2-methyl-1-propanol | - | - | Proper quantity | - | - | - | Proper quantity | - |
| | Ethanol | 5.0 | 5.0 | 5.0 | 5.0 | 5.0 | 5.0 | 5.0 | 5.0 |
| | De-ionized water | 34.4 | 34.4 | 34.4 | 34.4 | 34.4 | 34.4 | 34.4 | 34.4 |
| Touch | | | | | | | | | |
| Durability | | | | | | | | | |
| Hair washability | | | | | | | | | |
| Feel | | | | | | | | | |

Table 15

(Gel-like hair fixative) (Part)

| | | Comparative Examples | | |
|---|---|---|---|---|
| | | 9c | 10c | 11c |
| Component X | Thickening agent | 1.5 | 1.5 | 1.5 |
| | Triethanolamine | 1.1 | 1.1 | 1.1 |
| | Ethanol | 5.0 | 5.0 | 5.0 |
| | Deionized water | 50.0 | 50.0 | 50.0 |
| Component Y | Amphoteric urethane resin | 3.0 | 3.0 | 3.0 |
| | (Kind) | (a) | (b) | (c) |
| | Water-soluble resin | - | - | - |
| | (Kind) | - | - | - |
| | 2-amino-2-methyl-1-propanol | - | - | - |
| | Ethanol | 5.0 | 5.0 | 5.0 |
| | Deionized water | 34.4 | 34.4 | 34.4 |
| Touch | | | | |
| Durability | | | | |
| Hair washability | | | | |
| Feel | | | | |

It is clear from the above Table 11 to 15 that the Examples of the gel-like hair fixative have very good touch, good feel, and ensure excellent hair washability because of containing amphoteric urethane resin, and are high in curl retention and excellent in durability because of the water-soluble resin content.

In contrast, it is clear that the Comparative Examples 1c to 8c of the gel type hair fixative containing no amphoteric urethane resin have very bad touch, and that the Comparative Examples 9c to 11c of the gel type hair fixative containing no water-soluble resin are low in curl retention and poor in durability.

Hair cosmetics (pump spray hair fixative)

Examples 1d to 24d. Comparative Examples 1d to 11d

Each component shown in the following Table 16 to 20 was blended at the ratios shown therein and was mixed until becoming homogeneous to make the pump spray hair fixative.

The properties of the pump spray hair fixatives thus obtained in the Examples and Comparative Examples were evaluated in accordance with the standard for the aforementioned cosmetics for hair. The results are concurrently represented in the following Tables 16 to 20.

Table 16

(Pumping spray-type hair fixative) (Part)

| | Examples | | | | | | | |
|---|---|---|---|---|---|---|---|---|
| | 1d | 2d | 3d | 4d | 5d | 6d | 7d | 8d |
| Amphoteric urethane resin | 1.5 | 1.5 | 1.5 | 1.5 | 1.5 | 1.5 | 1.5 | 1.5 |
| (Kind) | (a) | (a) | (a) | (a) | (a) | (a) | (a) | (a) |
| Water-soluble resin | 1.5 | 1.5 | 1.5 | 1.5 | 1.5 | 1.5 | 1.5 | 1.5 |
| (Kind) | (1) | (2) | (3) | (4) | (5) | (6) | (7) | (8) |
| 2-amino-2-methyl-1-propanol | - | - | Proper quantity | - | - | - | Proper quantity | - |
| Dioctyl sodium sulfosuccinate | 0.3 | 0.3 | 0.3 | 0.3 | 0.3 | 0.3 | 0.3 | 0.3 |
| Ethanol | 10.0 | 10.0 | 10.0 | 10.0 | 10.0 | 10.0 | 10.0 | 10.0 |
| De-ionized water | 86.7 | 86.7 | 86.7 | 86.7 | 86.7 | 86.7 | 86.7 | 86.7 |
| Touch | | | | | | | | |
| Durability | | | | | | | | |
| Hair washability | | | | | | | | |
| Feel | | | | | | | | |

Table 17

(Pumping spray-type hair fixative) (Part)

| | Examples | | | | | | | |
|---|---|---|---|---|---|---|---|---|
| | 9d | 10d | 11d | 12d | 13d | 14d | 15d | 16d |
| Amphoteric urethane resin | 1.5 | 1.5 | 1.5 | 1.5 | 1.5 | 1.5 | 1.5 | 1.5 |
| (Kind) | (b) | (b) | (b) | (b) | (b) | (b) | (b) | (b) |
| Water-soluble resin | 1.5 | 1.5 | 1.5 | 1.5 | 1.5 | 1.5 | 1.5 | 1.5 |
| (Kind) | (1) | (2) | (3) | (4) | (5) | (6) | (7) | (8) |
| 2-amino-2-methyl-1-propanol | - | - | Proper quantity | - | - | - | Proper quantity | - |
| Dioctyl sodium sulfosuccinate | 0.3 | 0.3 | 0.3 | 0.3 | 0.3 | 0.3 | 0.3 | 0.3 |
| Ethanol | 10.0 | 10.0 | 10.0 | 10.0 | 10.0 | 10.0 | 10.0 | 10.0 |
| De-ionized water | 86.7 | 86.7 | 86.7 | 86.7 | 86.7 | 86.7 | 86.7 | 86.7 |
| Touch | | | | | | | | |
| Durability | | | | | | | | |
| Hair washability | | | | | | | | |
| Feel | | | | | | | | |

Table 18

(Pumping spray-type hair fixative) (Part)

| | Examples | | | | | | | |
|---|---|---|---|---|---|---|---|---|
| | 17d | 18d | 19d | 20d | 21d | 22d | 23d | 24d |
| Amphoteric urethane resin | 1.5 | 1.5 | 1.5 | 1.5 | 1.5 | 1.5 | 1.5 | 1.5 |
| (Kind) | (c) | (c) | (c) | (c) | (c) | (c) | (c) | (c) |
| Water-soluble resin | 1.5 | 1.5 | 1.5 | 1.5 | 1.5 | 1.5 | 1.5 | 1.5 |
| (Kind) | (1) | (2) | (3) | (4) | (5) | (6) | (7) | (8) |
| 2-amino-2-methyl-1-propanol | - | - | Proper quantity | - | - | - | Proper quantity | - |
| Dioctyl sodium sulfosuccinate | 0.3 | 0.3 | 0.3 | 0.3 | 0.3 | 0.3 | 0.3 | 0.3 |
| Ethanol | 10.0 | 10.0 | 10.0 | 10.0 | 10.0 | 10.0 | 10.0 | 10.0 |
| De-ionized water | 86.7 | 86.7 | 86.7 | 86.7 | 86.7 | 86.7 | 86.7 | 86.7 |
| Touch | | | | | | | | |
| Durability | | | | | | | | |
| Hair washability | | | | | | | | |
| Feel | | | | | | | | |

Table 19

(Pumping spray-type hair fixative) (Part)

| | Comparative Examples | | | | | | | |
|---|---|---|---|---|---|---|---|---|
| | 1d | 2d | 3d | 4d | 5d | 6d | 7d | 8d |
| Amphoteric urethane resin | - | - | - | - | - | - | - | - |
| (Kind) | - | - | - | - | - | - | - | - |
| Water-soluble resin | 3.0 | 3.0 | 3.0 | 3.0 | 3.0 | 3.0 | 3.0 | 3.0 |
| (Kind) | (1) | (2) | (3) | (4) | (5) | (6) | (7) | (8) |
| 2-amino-2-methyl-1-propanol | - | - | Proper quantity | - | - | - | Proper quantity | - |
| Dioctyl sodium sulfosuccinate | 0.3 | 0.3 | 0.3 | 0.3 | 0.3 | 0.3 | 0.3 | 0.3 |
| Ethanol | 10.0 | 10.0 | 10.0 | 10.0 | 10.0 | 10.0 | 10.0 | 10.0 |
| De-ionized water | 86.7 | 86.7 | 86.7 | 86.7 | 86.7 | 86.7 | 86.7 | 86.7 |
| Touch | | | | | | | | |
| Durability | | | | | | | | |
| Hair washability | | | | | | | | |
| Feel | | | | | | | | |

Table 20

(Pumping spray-type hair fixative) (Part)

| | Comparative Examples | | |
|---|---|---|---|
| | 9d | 10d | 11d |
| Amphoteric urethane resin | 3.0 | 3.0 | 3.0 |
| (Kind) | (a) | (b) | (c) |
| Water-soluble resin | - | - | - |
| (Kind) | - | - | - |
| 2-amino-2-methyl-1-propanol | - | - | - |
| Dioctyl sodium sulfosuccinate | 0.3 | 0.3 | 0.3 |
| Ethanol | 10.0 | 10.0 | 10.0 |
| De-ionized water | 86.7 | 86.7 | 86.7 |
| Touch | | | |
| Durability | | | |
| Hair washability | | | |
| Feel | | | |

It is clear from the above Table 16 to 20 that the Examples of the pump spray hair fixative have very good touch, good feel, and ensure excellent hair washability because of containing amphoteric urethane resin, and are high in curl retention and excellent in durability because of containing water-soluble resin.

In contrast, it is clear that the Comparative Examples 1d to 8d of the pump spray hair fixative containing no amphoteric urethane resin have very bad touch, and that the Comparative Examples 9d to 11d of the pump spray hair fixative containing no water-soluble resin are low in curl retention and poor in durability.

Skin care cosmetics (skin care lotion)

Examples 1e to 4e. Comparative Examples 1e to 5e

The X component was obtained by admixing each material of the X component shown in Table 21 and Table 22 mentioned later at the ratios shown therein and heating to 80°C. Also, the Y component was obtained by admixing each material of the Y components shown in the said Tables at the ratios shown therein and heating to 80°C. Subsequently, the above-mentioned X and Y components were mixed and agitated at 80 °C for 30 minutes, and then a thickening agent was added to the mixture and agitated until becoming homogeneous. After that, the (XY) mixture was cooled down to 40°C to make the skin care lotion.

The properties of the skin care lotion thus obtained in the Examples and Comparative Examples were evaluated in accordance with the following standards. The results are concurrently represented in the following Tables 21 to 22.

Touch

10 panelists conducted practical application tests for evaluation of the touch that the test pieces should be inherently endowed with as skin care cosmetics. The evaluation standard is as follows:

: 9 ~ 10 persons whose skin felt smooth and soft without feeling tight.
: 6 ~ 8 persons whose skin felt smooth and soft without feeling tight.

: 2 ~ 5 persons whose skin felt smooth and soft without feeling tight.
×: 0 ~ 1 person whose skin felt smooth and soft without feeling tight.

Durability

10 panelists conducted practical application tests for evaluation of the durability that the test pieces should be inherently endowed with as skin care cosmetics. The evaluation standard is as follows:

: 9 ~ 10 persons who felt the applied cosmetics very lasting after lapse of 6 hours.
: 6 ~ 8 persons who felt the applied cosmetics very lasting after lapse of 6 hours.
: 2 ~ 5 persons who felt the applied cosmetics very lasting after lapse of 6 hours.
×: 0 ~ 1 person who felt the applied cosmetics very lasting after lapse of 6 hours.

Table 21 (Skin care lotion) (Part)

| | | Examples | | | |
|---|---|---|---|---|---|
| | | 1e | 2e | 3e | 4e |
| Component X | Octyl methoxycinnamate | 7.5 | 7.5 | 7.5 | 7.5 |
| | Ether polyoxystearate | 1.0 | 1.0 | 1.0 | 1.0 |
| | Emulsifiable glyceryl stearate | 1.0 | 1.0 | 1.0 | 1.0 |
| | Stearic acid | 1.5 | 1.5 | 1.5 | 1.5 |
| | Mixture of titanium dioxide and benzoic $C_{12-15}$ alkyl | 1.7 | 1.7 | 1.7 | 1.7 |
| | Polyoxyethylene-additive dimethycone | 0.5 | 0.5 | 0.5 | 0.5 |
| Component Y | Amphoteric urethane resin | 0.5 | 0.5 | 0.5 | 0.5 |
| | (Kind) | (a) | (a) | (a) | (a) |
| | Water-soluble resin | 0.5 | 0.5 | 0.5 | 0.5 |
| | (Kind) | (1) | (3) | (5) | (7) |
| | De-ionized water | 61.8 | 61.8 | 61.8 | 61.8 |
| | Triethanolamine (99%) | 4.0 | 4.0 | 4.0 | 4.0 |
| | Antiseptic agent | Proper quantity | Proper quantity | Proper quantity | Proper quantity |
| | Thickening agent (2%) | 20.0 | 20.0 | 20.0 | 20.0 |
| | 2-amino-2-methyl-1-propanol | - | Proper quantity | - | Proper quantity |
| Touch | | | | | |
| Durability | | | | | |

Table 22

(Skin care lotion) (Part)

| | | Comparative Examples | | | | |
|---|---|---|---|---|---|---|
| | | 1e | 2e | 3e | 4e | 5e |
| Component X | Octyl methoxycinnamate | 7.5 | 7.5 | 7.5 | 7.5 | 7.5 |
| | Polyoxystearate ether | 1.0 | 1.0 | 1.0 | 1.0 | 1.0 |
| | Emulsifiable glyceryl stearate | 1.0 | 1.0 | 1.0 | 1.0 | 1.0 |
| | Stearic acid | 1.5 | 1.5 | 1.5 | 1.5 | 1.5 |
| | Mixture of titanium dioxide and $C_{12-15}$ alkyl benzoate | 1.7 | 1.7 | 1.7 | 1.7 | 1.7 |
| | Polyoxyethylene-additive Dimethycone | 0.5 | 0.5 | 0.5 | 0.5 | 0.5 |
| Component Y | Amphoteric urethane resin | - | - | - | - | 1.0 |
| | (Kind) | - | - | - | - | (a) |
| | Water-soluble resin | 1.0 | 1.0 | 1.0 | 1.0 | - |
| | (Kind) | (1) | (3) | (5) | (7) | - |
| | De-ionized water | 61.8 | 61.8 | 61.8 | 61.8 | 61.8 |
| | Triethanolamine (99%) | 4.0 | 4.0 | 4.0 | 4.0 | 4.0 |
| | Antiseptic agent | Proper quantity | Proper quantity | Proper quantity | Proper quantity | Proper quantity |
| | Thickening agent (2%) | 20.0 | 20.0 | 20.0 | 20.0 | 20.0 |
| | 2-amino-2-methyl-1-propanol | - | Proper quantity | - | Proper quantity | - |
| Touch | | | | | | |
| Durability | | | | | | |

It is clear from the above Table 21 and Table 22 that the Examples of the skin care lotion have very good touch because of the amphoteric urethane resin content and are excellent in durability because of the water-soluble resin content.

In contrast, the Comparative Examples 1e to 4e of the skin care lotion containing no amphoteric urethane resin have very bad touch, and the compared example 5e of the skin care lotion containing no water-soluble resin is poor in durability.

Skin care cosmetics] (shaving cream)

Examples 1f to 4f. Comparative Examples 1f to 5f

The X component was obtained by admixing each material of the X components shown in the following Table 23 and Table 24 at the ratios shown therein and heating to 80°C. Also, the Y component was obtained by admixing each material of the Y components at the ratios shown in the said Tables and heating to 80°C. Subsequently, the above-mentioned X and Y components were mixed at 80°C and cooled down to 40°C, and then proper amounts of antiseptic agent and perfume were added to the mixture to make the shaving cream agent.

The properties of the shaving cream agent thus obtained in the Examples and Comparative Examples were evaluated in accordance with the standard for the aforementioned skin care cosmetics. The results are concurrently represented in the following Tables 23 and Table 24.

Table 23

(Shaving cream agent) (Part)

| | | Examples | | | |
|---|---|---|---|---|---|
| | | 1f | 2f | 3f | 4f |
| Component X | Stearic acid | 8.0 | 8.0 | 8.0 | 8.0 |
| | Mineral oil | 2.0 | 2.0 | 2.0 | 2.0 |
| | Isopropyl myristate | 2.0 | 2.0 | 2.0 | 2.0 |
| | Glyceryl stearate | 0.5 | 0.5 | 0.5 | 0.5 |
| Component Y | Amphoteric urethane resin | 0.3 | 0.3 | 0.3 | 0.3 |
| | (Kind) | (a) | (a) | (a) | (a) |
| | Water-soluble resin | 0.3 | 0.3 | 0.3 | 0.3 |
| | (Kind) | (1) | (3) | (5) | (7) |
| | De-ionized water | 72.7 | 72.7 | 72.7 | 72.7 |
| | Thickening agent (2%) | 10.0 | 10.0 | 10.0 | 10.0 |
| | Triethanolamine (99%) | 4.2 | 4.2 | 4.2 | 4.2 |
| | 2-amino-2-methyl-1-propanol | - | Proper quantity | - | Proper quantity |
| Touch | | | | | |
| Durability | | | | | |

Table 24

(Shaving cream agent) (Part)

| | | Comparative Examples | | | | |
|---|---|---|---|---|---|---|
| | | 1f | 2f | 3f | 4f | 5f |
| Component X | Stearic acid | 8.0 | 8.0 | 8.0 | 8.0 | 8.0 |
| | Mineral oil | 2.0 | 2.0 | 2.0 | 2.0 | 2.0 |
| | Isopropyl myristate | 2.0 | 2.0 | 2.0 | 2.0 | 2.0 |
| | Glyceryl stearate | 0.5 | 0.5 | 0.5 | 0.5 | 0.5 |
| Component Y | Amphoteric urethane resin | - | - | - | - | 0.6 |
| | (Kind) | - | - | - | - | (a) |
| | Water-soluble resin | 0.6 | 0.6 | 0.6 | 0.6 | - |
| | (Kind) | (1) | (3) | (5) | (7) | - |
| | De-ionized water | 72.7 | 72.7 | 72.7 | 72.7 | 72.7 |
| | Thickening agent (2%) | 10.0 | 10.0 | 10.0 | 10.0 | 10.0 |
| | Triethanolamine (99%) | 4.2 | 4.2 | 4.2 | 4.2 | 4.2 |
| | 2-amino-2-methyl-1-propanol | - | Proper quantity | - | Proper quantity | - |
| Touch | | | | | | |
| Durability | | | | | | |

It is clear from the above Table 23 and Table 24 that the Examples of the shaving cream agent have very good touch because of the amphoteric urethane resin content, and are excellent in durability because of the water-soluble resin content.

In contrast, the Comparative Examples 1f to 4f of the shaving cream agent containing no amphoteric urethane resin have very bad touch, and the Compared Example 5f of the shaving cream agent containing no water-soluble resin is poor in durability.

Skin care cosmetics (sunscreen lotion)

Examples 1g to 4g. Comparative Examples 1g to 5g

The X component was obtained by admixing each material of the X components shown in the following Table 25 and Table 26 at the ratios shown therein and heating to 80°C. Also, the Y component was obtained by admixing each material of the Y components at the ratios shown in the said Tables and heating to 80°C. Subsequently, the above-mentioned X and Y components were mixed at 80°C to make the sunscreen lotion.

The properties of the sunscreen lotion thus obtained in the Examples and Comparative Examples were evaluated in accordance with the standard for the aforementioned skin care cosmetics. The results are concurrently represented in the following Tables 25 and Table 26.

Table 25

(Sunscreen lotion) (Part)

| | | Examples | | | |
|---|---|---|---|---|---|
| | | 1g | 2g | 3g | 4g |
| Component X | Octyl methoxycinnamate | 7.5 | 7.5 | 7.5 | 7.5 |
| | Octyl palmitate | 5.0 | 5.0 | 5.0 | 5.0 |
| | Cetyl alcohol | 1.0 | 1.0 | 1.0 | 1.0 |
| | Polyethylene glycol monostearate | 1.5 | 1.5 | 1.5 | 1.5 |
| | Poly (oxyethylene oxypropylene) methylpolysiloxane copolymer | 1.0 | 1.0 | 1.0 | 1.0 |
| | Dimethylstearylamine | 2.0 | 2.0 | 2.0 | 2.0 |
| Component Y | Amphoteric urethane resin | 1.0 | 1.0 | 1.0 | 1.0 |
| | (Kind) | (a) | (a) | (a) | (a) |
| | Water-soluble resin | 1.0 | 1.0 | 1.0 | 1.0 |
| | (Kind) | (1) | (3) | (5) | (7) |
| | Purified water | 69.05 | 69.05 | 69.05 | 69.05 |
| | Triethanolamine (99%) | 0.7 | 0.7 | 0.7 | 0.7 |
| | Thickening agent (2%) | 10.0 | 10.0 | 10.0 | 10.0 |
| | Antiseptic agent | 0.25 | 0.25 | 0.25 | 0.25 |
| | 2-amino-2-methyl-1-propanol | - | Proper quantity | - | Proper quantity |
| Touch | | | | | |
| Durability | | | | | |

Table 26

(Sunscreen lotion) (Part)

| | | Comparative Example | | | | |
|---|---|---|---|---|---|---|
| | | 1g | 2g | 3g | 4g | 5g |
| Component X | Octyl methoxycinnamate | 7.5 | 7.5 | 7.5 | 7.5 | 7.5 |
| | Octyl palmitate | 5.0 | 5.0 | 5.0 | 5.0 | 5.0 |
| | Cetyl alcohol | 1.0 | 1.0 | 1.0 | 1.0 | 1.0 |
| | Polyethylene glycol monostearate | 1.5 | 1.5 | 1.5 | 1.5 | 1.5 |
| | Poly (oxyethylene oxypropylene) methylpolysiloxane copolymer | 1.0 | 1.0 | 1.0 | 1.0 | 1.0 |
| | Dimethylstearylamine | 2.0 | 2.0 | 2.0 | 2.0 | 2.0 |
| Component Y | Amphoteric urethane resin | - | - | - | - | 2.0 |
| | (Kind) | - | - | - | - | (a) |
| | Water-soluble resin | 2.0 | 2.0 | 2.0 | 2.0 | - |
| | (Kind) | (1) | (3) | (5) | (7) | - |
| | Purified water | 69.05 | 69.05 | 69.05 | 69.05 | 69.05 |
| | Triethanolamine (99%) | 0.7 | 0.7 | 0.7 | 0.7 | 0.7 |
| | Thickening agent (2%) | 10.0 | 10.0 | 10.0 | 10.0 | 10.0 |
| | Antiseptic agent | 0.25 | 0.25 | 0.25 | 0.25 | 0.25 |
| | 2-amino-2-methyl-1-propanol | - | Proper quantity | - | Proper quantity | - |
| Touch | | | | | | |
| Durability | | | | | | |

It is clear from the above Table 25 and Table 26 that the Examples of the sunscreen lotion have very good touch because of the amphoteric urethane resin content, and are excellent in durability because of the water-soluble resin content.

In contrast, the Comparative Examples 1g to 4g of the sunscreen lotion containing no amphoteric urethane resin have very bad touch, and that the compared example 5g of the sunscreen lotion containing no water-soluble resin is poor in durability.

Make-up cosmetics (nail polish)

Examples 1h to 4h. Comparative Example 1h to 5h

As shown in Table 27 and Table 28 mentioned later, the pigment was dispersed in de-ionized water at the ratios shown in the said Tables, and then the other components were added at the ratios shown therein. After that, the solution was uniformly agitated, mixed, and deaerated to make the nail polish.

The properties of the nail polish thus obtained in the Examples and Comparative Examples were evaluated

in accordance with the following standards. The results are concurrently represented in the following Tables 27 and Table 28.

Touch

10 panelists conducted practical application tests for evaluation of the touch that the test pieces should be inherently endowed with as make-up cosmetics. The evaluation standard is as follows:

: 9 ~ 10 persons felt, the applied area smooth and soft without feeling tight.
: 6 ~ 8 persons felt the applied area smooth and soft without feeling tight.
: 2 ~ 5 persons felt the applied area smooth and soft without feeling tight.
×: 0 ~ 1 person felt the applied area smooth and soft without feeling tight.

Durability

10 panelists conducted practical application tests for evaluation of the durability that the test pieces should be inherently endowed with as make-up cosmetics. The evaluation standard is as follows:

: 9 ~ 10 persons who felt the applied cosmetics very lasting after lapse of 6 hours.
: 6 ~ 8 persons who felt the applied cosmetics very lasting after lapse of 6 hours.
: 2 ~ 5 persons who felt the applied cosmetics very lasting after lapse of 6 hours.
×: 0 ~ 1 person who felt the applied cosmetics very lasting after lapse of 6 hours.

Table 27

(Nail polish) (Part)

| | | Examples | | | |
|---|---|---|---|---|---|
| | | 1h | 2h | 3h | 4h |
| Aqueous phase | Amphoteric urethane resin | 5.0 | 5.0 | 5.0 | 5.0 |
| | (Kind) | (a) | (a) | (a) | (a) |
| | Water-soluble resin | 5.0 | 5.0 | 5.0 | 5.0 |
| | (Kind) | (1) | (3) | (5) | (7) |
| | De-ionized water | 86.6 | 86.6 | 86.6 | 86.6 |
| | Bentonite | 0.6 | 0.6 | 0.6 | 0.6 |
| | 2-amino-2-methyl-1-propanol | - | Proper quantity | - | Proper quantity |
| Pigment | | 2.5 | 2.5 | 2.5 | 2.5 |
| Others | Perfume | 0.1 | 0.1 | 0.1 | 0.1 |
| | Antiseptic agent | 0.1 | 0.1 | 0.1 | 0.1 |
| | Silicone antifoamer | 0.1 | 0.1 | 0.1 | 0.1 |
| Touch | | | | | |
| Durability | | | | | |

Table 28

(Nail polish) (Part)

| | | Comparative Examples | | | | |
|---|---|---|---|---|---|---|
| | | 1h | 2h | 3h | 4h | 5h |
| Aqueous phase | Amphoteric urethane resin | - | - | - | - | 10.0 |
| | (Kind) | - | - | - | - | (a) |
| | Water-soluble resin | 10.0 | 10.0 | 10.0 | 10.0 | - |
| | (Kind) | (1) | (3) | (5) | (7) | - |
| | De-ionized water | 86.6 | 86.6 | 86.6 | 86.6 | 86.6 |
| | Bentonite | 0.6 | 0.6 | 0.6 | 0.6 | 0.6 |
| | 2-amino-2-methyl-1-propanol | - | Proper quantity | - | Proper quantity | - |
| Pigment | | 2.5 | 2.5 | 2.5 | 2.5 | 2.5 |
| Others | Perfume | 0.1 | 0.1 | 0.1 | 0.1 | 0.1 |
| | Antiseptic agent | 0.1 | 0.1 | 0.1 | 0.1 | 0.1 |
| | Silicone antifoamer | 0.1 | 0.1 | 0.1 | 0.1 | 0.1 |
| Touch | | | | | | |
| Durability | | | | | | |

It is clear from the above Table 27 and Table 28 that the Examples of the nail polish have very good touch because of the amphoteric urethane resin content, and are excellent in durability because of the water-soluble resin content.

In contrast, the Comparative Example 1 h to 4h of the nail polish containing no amphoteric urethane resin have very bad touch, and that the Ccomparative Example 5h of the nail polish containing no water-soluble resin is poor in durability.

Make-up cosmetics (mascara)

Examples 1i to 4i. Comparative Examples 1i to 5i

As shown in the following Table 29 and Table 30, propylene glycol, triethanolamine, thickening agent, antiseptic agent, and 2-amino-2-methyl-1- propanol were blended in refined water at the ratios shown in the said Tables and dissolved at 80°C, and then pigment was dispersed therein to make an aqueous phase. Subsequently, each material of the Z component was blended at the ratios shown in the said Tables, and then dissolved at 80°C to make an oil phase. The oil phase was applied into the aqueous phase and emulsified by using a homogenizing mixer. After that, each material of the Y component was slowly applied into the homogeneous emulsion at the ratios shown in the said Tables and agitated by using a homogenizing mixer, and then the emulsion was cooled down to normal temperature

to make the mascara.

The properties of the mascara thus obtained in the Examples and Comparative Examples were evaluated in accordance with the standard for the aforementioned make-up cosmetics. The results are concurrently represented in the following Tables 29 and Table 30.

Table 29

(Mascara) (Part)

| | | Examples | | | |
|---|---|---|---|---|---|
| | | 1i | 2i | 3i | 4i |
| Component X | Propyleneglycol | 5.0 | 5.0 | 5.0 | 5.0 |
| | Triethanolamine | 1.0 | 1.0 | 1.0 | 1.0 |
| | Thickening agent (2%) | 10.0 | 10.0 | 10.0 | 10.0 |
| | Antiseptic agent | 0.5 | 0.5 | 0.5 | 0.5 |
| | Pigment | 10.0 | 10.0 | 10.0 | 10.0 |
| | Purified water | 55.5 | 55.5 | 55.5 | 55.5 |
| | 2-amino-2-methyl-1-propanol | - | Proper quantity | - | Proper quantity |
| Component Y | Amphoteric urethane resin | 3.0 | 3.0 | 3.0 | 3.0 |
| | (Kind) | (a) | (a) | (a) | (a) |
| | Water-soluble resin | 3.0 | 3.0 | 3.0 | 3.0 |
| | (Kind) | (1) | (3) | (5) | (7) |
| Compo - nent Z | Stearic acid | 6.0 | 6.0 | 6.0 | 6.0 |
| | Beeswax | 6.0 | 6.0 | 6.0 | 6.0 |
| Touch | | | | | |
| Durability | | | | | |

Table 30

(Mascara) (Part)

| | | Comparative Examples | | | | |
|---|---|---|---|---|---|---|
| | | 1i | 2i | 3i | 4i | 5i |
| Component X | Propyleneglycol | 5.0 | 5.0 | 5.0 | 5.0 | 5.0 |
| | Triethanolamine | 1.0 | 1.0 | 1.0 | 1.0 | 1.0 |
| | Thickening agent (2%) | 10.0 | 10.0 | 10.0 | 10.0 | 10.0 |
| | Antiseptic agent | 0.5 | 0.5 | 0.5 | 0.5 | 0.5 |
| | Pigment | 10.0 | 10.0 | 10.0 | 10.0 | 10.0 |
| | Purified water | 55.5 | 55.5 | 55.5 | 55.5 | 55.5 |
| | 2-amino-2-methyl-1-propanol | - | Proper quantity | - | Proper quantity | - |
| Component Y | Amphoteric urethane resin | - | - | - | - | 6.0 |
| | (Kind) | - | - | - | - | (a) |
| | Water-soluble resin | 6.0 | 6.0 | 6.0 | 6.0 | - |
| | (Kind) | (1) | (3) | (5) | (7) | - |
| Compo-nent Z | Stearic acid | 6.0 | 6.0 | 6.0 | 6.0 | 6.0 |
| | Beeswax | 6.0 | 6.0 | 6.0 | 6.0 | 6.0 |
| Touch | | | | | | |
| Durability | | | | | | |

It is clear from the above Table 29 and Table 30 that the Examples of the mascara have very good touch because of the amphoteric urethane resin content, and are excellent in durability because of the water-soluble resin content.

In contrast, the Comparative Example 1i to 4i of the mascara containing no amphoteric urethane resin have very bad touch, and the Comparative Example 5i of the mascara containing no water-soluble resin is poor in durability.

Make-up cosmetics (foundation)

Examples 1j to 4j, Comparative Examples 1j to 5j

(1) Preparation of pigment

Each component shown in the following Table 31 and Table 32 was mixed at the ratios shown therein, and then milled through a grinder to prepare the pigment.

(2) Preparation of aqueous phase

After heating de-ionized water to 70°C, bentonite was added to the water for the purpose of swelling. Subsequently, sodium carboxymethyl cellulose previously dispersed in propylene glycol was added and dissolved therein. Subsequently, triethanol amine, paraoxy methyl bensoate, 2-amino-2-methyl-1-propanol, and at least one of amphoteric urethane resin and water-soluble resin were added and dissolved therein to prepare the aqueous phase.

(3) Preparation of oil phase

Each component shown in the following Table 31 and Table 32 was mixed at the ratios shown therein, and then heated at 70°C to 80°C and dissolved to prepare the oil phase.

(4) Preparation of pigment dispersed solution

The aforementioned pigment was added to the above aqueous phase while agitating, and then treated through a colloid mill to prepare the pigment dispersed solution.

(5) Emulsification

After heating the above pigment dispersed solution at 75°C and the oil phase at 80°C respectively, the said oil phase was added to the said pigment dispersed solution while agitating, and then cooled to 45°C at which perfume was added, continuously agitated and cooled until reaching room temperature to prepare the foundation.

The properties of the foundation thus obtained in the Examples and Comparative Examples were evaluated in accordance with the standard for the aforementioned make-up cosmetics. The results are concurrently represented in the following Tables 31 and Table 32.

Table 31

(Foundation) (Part)

| | | Examples | | | |
|---|---|---|---|---|---|
| | | 1j | 2j | 3j | 4j |
| Oil phase | Stearic acid | 2.4 | 2.4 | 2.4 | 2.4 |
| | Propylene glycol monostearate | 2.0 | 2.0 | 2.0 | 2.0 |
| | Cetostearyl alcohol | 0.2 | 0.2 | 0.2 | 0.2 |
| | Liquid lanolin | 2.0 | 2.0 | 2.0 | 2.0 |
| | Liquid paraffin | 3.0 | 3.0 | 3.0 | 3.0 |
| | Isopropyl myristate | 8.5 | 8.5 | 8.5 | 8.5 |
| | Propyl paraoxybenzoate | Proper quantity | Proper quantity | Proper quantity | Proper quantity |
| Aqueous phase | Amphoteric urethane resin | 0.3 | 0.3 | 0.3 | 0.3 |
| | (Kind) | (a) | (a) | (a) | (a) |
| | Water-soluble resin | 0.3 | 0.3 | 0.3 | 0.3 |
| | (Kind) | (1) | (3) | (5) | (7) |
| | De-ionized water | 63.5 | 63.5 | 63.5 | 63.5 |
| | Carboxymethyl cellulose sodium | 0.2 | 0.2 | 0.2 | 0.2 |
| | Bentonite | 0.5 | 0.5 | 0.5 | 0.5 |
| | Propylene glycol | 4.0 | 4.0 | 4.0 | 4.0 |
| | Triethanolamine | 1.1 | 1.1 | 1.1 | 1.1 |
| | Methyl paraoxybenzoate | Proper quantity | Proper quantity | Proper quantity | Proper quantity |
| | 2-amino-2-methyl-1-propanol | - | Proper quantity | - | Proper quantity |
| Pig-ment | Titanium oxide | 8.0 | 8.0 | 8.0 | 8.0 |
| | Talc | 4.0 | 4.0 | 4.0 | 4.0 |
| | Coloring pigment | Proper Quantity | Proper Quantity | Proper Quantity | Proper Quantity |
| Perfume | | Proper quantity | Proper quantity | Proper quantity | Proper quantity |
| Touch | | | | | |
| Durability | | | | | |

Table 32

(Foundation) (Part)

| | | Comparative Examples | | | | |
|---|---|---|---|---|---|---|
| | | 1j | 2j | 3j | 4j | 5j |
| Oil phase | Stearic acid | 2.4 | 2.4 | 2.4 | 2.4 | 2.4 |
| | Propylene glycol monostearate | 2.0 | 2.0 | 2.0 | 2.0 | 2.0 |
| | Cetostearyl alcohol | 0.2 | 0.2 | 0.2 | 0.2 | 0.2 |
| | Liquid lanolin | 2.0 | 2.0 | 2.0 | 2.0 | 2.0 |
| | Liquid paraffin | 3.0 | 3.0 | 3.0 | 3.0 | 3.0 |
| | Isopropyl myristate | 8.5 | 8.5 | 8.5 | 8.5 | 8.5 |
| | Propyl paraoxybenzonate | Proper quantity | Proper quantity | Proper quantity | Proper quantity | Proper quantity |
| Aqueous phase | Amphoteric urethane resin | - | - | - | - | 0.6 |
| | (Kind) | - | - | - | - | (a) |
| | Water-soluble resin | 0.6 | 0.6 | 0.6 | 0.6 | - |
| | (Kind) | (1) | (3) | (5) | (7) | - |
| | De-ionized water | 63.5 | 63.5 | 63.5 | 63.5 | 63.5 |
| | Carboxymethyl cellulose sodium | 0.2 | 0.2 | 0.2 | 0.2 | 0.2 |
| | Bentonite | 0.5 | 0.5 | 0.5 | 0.5 | 0.5 |
| | Propyleneglycol | 4.0 | 4.0 | 4.0 | 4.0 | 4.0 |
| | Triethanolamine | 1.1 | 1.1 | 1.1 | 1.1 | 1.1 |
| | Methyl paraoxybenzoate | Proper quantity | Proper quantity | Proper quantity | Proper quantity | Proper quantity |
| | 2-amino-2-methyl-1-propanol | - | Proper quantity | - | Proper quantity | - |
| Pig-ment | Titanium oxide | 8.0 | 8.0 | 8.0 | 8.0 | 8.0 |
| | Talc | 4.0 | 4.0 | 4.0 | 4.0 | 4.0 |
| | Coloring pigment | Proper Quantity | Proper Quantity | Proper Quantity | Proper Quantity | Proper Quantity |
| Perfume | | Proper quantity | Proper quantity | Proper quantity | Proper quantity | Proper quantity |
| Touch | | | | | | |
| Durability | | | | | | |

It is clear from the above Table 31 and Table 32 that the Examples of the foundation have very good touch because of the amphoteric urethane resin content, and are excellent in durability because of the water-soluble resin content.

In contrast, the Comparative Examples 1j to 4j of the foundation containing no amphoteric urethane resin have very bad touch, and the compared example 5j of the foundation containing no water-soluble resin is poor in durability.

Effects of the Invention

As described above, in the cosmetics of the present invention, using as a base resin amphoteric urethane resin containing carboxyl group(s) and tertiary amino group(s) in one molecule, originally antithetic physical properties such as setting ability and touch, combing ability and resistance to flaking are compatible with each other by the elasticity and toughness that the urethane resin possesses. Furthermore, as against neutral water, the said cosmetics ensure excellent water resistance because of the ion bond(s) between carboxyl group(s) and tertiary amino group(s). On the other hand, as against surface-active agent solution such as shampoo, etc., the said cosmetics ensure excellent hair washability with dissociation of the said ion bond(s), and at the same time, the above-mentioned cationic tertiary amino group(s) interacts on the surfaces of the negatively charged hair to ensure excellent adhesion. Moreover, the cosmetics of the present invention use both water-soluble resin and amphoteric urethane resin, therefore the problem of the durability which is a weak point of amphoteric urethane resin can be solved by the use of water-soluble resin, and also the problem of the touch which is a weak point of water-soluble resin can be solved by the use of amphoteric urethane resin, and thereby the said cosmetics are provided with antithetic physical properties such as touch and durability which are required for cosmetics.

Additionally, the durability may be further improved by using nonionic resin, anionic resin ,cationic resin or amphoteric resin as said water-soluble resin.

The introduction into the structure of the said amphoteric urethane resin of the structural unit which may be derived from ethylene oxide as nonionic phydrophilic constituent may provide sufficient hydrophilic nature, which ensures improvement of hair washability especially when it is used as hair cosmetic material.

Additionally, the introduction of polysiloxane bond(s) into the structure of the said amphoteric urethane resin can improve the feel which may be felt especially when it is used as hair cosmetic material.

## Claims

1. A cosmetic composition comprising an amphoteric urethane resin having at least one carboxyl group and at least one tertiary amino group in one molecule, and a water-soluble resin.

2. The cosmetic composition of claim 1, wherein the water-soluble resin is a nonionic resin.

3. The cosmetic composition of claim 1, wherein the water-soluble resin is an anionic resin.

4. The cosmetic composition of claim 1, wherein the water-soluble resin is a cationic resin.

5. The cosmetic composition of claim 1, wherein the water-soluble resin is an amphoteric resin.

6. The cosmetic composition of any one of claims 1 to 5, wherein the amphoteric urethane resin has in its structure a structural unit that is derived from ethylene oxide.

7. The cosmetic composition of any one of claims 1 to 5, wherein the amphoteric urethane resin has in its structure at least one polysiloxane bond.

8. The cosmetic composition of any one of claims 1 to 7, wherein the amphoteric urethane resin is an aqueous liquid.

9. The cosmetic composition of any one of claims 1 to 8, wherein the composition is selected from the group consisting of a hair cosmetic, a skin care cosmetic and a make-up cosmetic.

## Patentansprüche

1. Kosmetikzusammensetzung, umfassend ein amphoteres Urethanharz, das mindestens eine Carboxylgruppe und mindestens eine tertiäre Aminogruppe in einem Molekül hat, und ein wasserlösliches Harz.

2. Kosmetikzusammensetzung nach Anspruch 1, wobei das wasserlösliche Harz ein nichtionisches Harz ist.

3. Kosmetikzusammensetzung nach Anspruch 1, wobei das wasserlösliche Harz ein anionisches Harz ist.

4. Kosmetikzusammensetzung nach Anspruch 1, wobei das wasserlösliche Harz ein kationisches Harz ist.

5. Kosmetikzusammensetzung nach Anspruch 1, wobei das wasserlösliche Harz ein amphoteres Harz ist.

6. Kosmetikzusammensetzung nach einem der Ansprüche 1 bis 5, wobei das amphotere Urethanharz in seiner Struktur eine Struktureinheit hat, die von Ethylenoxid abgeleitet ist.

7. Kosmetikzusammensetzung nach einem der Ansprüche 1 bis 5, wobei das amphotere Urethanharz mindestens eine Polysiloxanbindung in seiner Struktur hat.

8. Kosmetikzusammensetzung nach einem der Ansprüche 1 bis 7, wobei das amphotere Urethanharz eine wässrige Flüssigkeit ist.

9. Kosmetikzusammensetzung nach einem der Ansprüche 1 bis 8, wobei die Zusammensetzung aus der Gruppe bestehend aus einem Haarkosmetikum, einem Hautpflegekosmetikum und einem Make-up-Kosmetikum ausgewählt ist.

**Revendications**

1. Composition cosmétique comprenant une résine d'uréthanne amphotère ayant au moins un groupe carboxyle et au moins un groupe amino tertiaire dans une molécule, et une résine soluble dans l'eau.

2. Composition cosmétique selon la revendication 1, dans laquelle la résine soluble dans l'eau est une résine non ionique.

3. Composition cosmétique selon la revendication 1, dans laquelle la résine soluble dans l'eau est une résine anionique.

4. Composition cosmétique selon la revendication 1, dans laquelle la résine soluble dans l'eau est une résine cationique.

5. Composition cosmétique selon la revendication 1, dans laquelle la résine soluble dans l'eau est une résine amphotère.

6. Composition cosmétique selon l'une quelconque des revendications 1 à 5, dans laquelle la résine d'uréthanne amphotère a une unité structurale qui est dérivée d'oxyde d'éthylène dans sa structure.

7. Composition cosmétique selon l'une quelconque des revendications 1 à 5, dans laquelle la résine d'uréthanne amphotère a au moins une liaison polysiloxane dans sa structure.

8. Composition cosmétique selon l'une quelconque des revendications 1 à 7, dans laquelle la résine d'uréthanne amphotère est un liquide aqueux.

9. Composition cosmétique selon l'une quelconque des revendications 1 à 8, ladite composition étant choisie dans l'ensemble constitué d'un cosmétique capillaire, d'un cosmétique de soins cutanés et d'un cosmétique de maquillage.